(19) Europäisches Patentamt
European Patent Office
Office européen des brevets

(11) EP 4 306 584 A1

(12) EUROPEAN PATENT APPLICATION

(43) Date of publication:
**17.01.2024 Bulletin 2024/03**

(21) Application number: **22184110.9**

(22) Date of filing: **11.07.2022**

(51) International Patent Classification (IPC):
***C08J 11/08*** *(2006.01)* ***C08J 11/12*** *(2006.01)*
***C08J 11/14*** *(2006.01)*

(52) Cooperative Patent Classification (CPC):
**C08J 11/08; C08J 11/12; C08J 11/14;**
C07D 201/12; C08J 2375/08; C08J 2377/02;
C08J 2475/08; C08J 2477/02

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **CAP III B.V.**
**6129 EL Urmond (NL)**

(72) Inventors:
- **Verduyckt, Jasper**
  **6129 EL Urmond (NL)**
- **Groothaert, Marijke Hilde Leen**
  **6129 EL Urmond (NL)**
- **van Heijningen, Niek**
  **6129 EL Urmond (NL)**
- **Westerhof, Jarno Martijn**
  **6129 EL Urmond (NL)**
- **Markusse, Abraham Peter**
  **6129 EL Urmond (NL)**
- **Roos, Peter**
  **6129 EL Urmond (NL)**
- **Cuiper, Anna Dite**
  **6129 EL Urmond (NL)**
- **Murphy, Kate Emily**
  **6129 EL Urmond (NL)**
- **Tinge, Johan Thomas**
  **6129 EL Urmond (NL)**

(74) Representative: **Cohausz & Florack**
**Patent- & Rechtsanwälte**
**Partnerschaftsgesellschaft mbB**
**Bleichstraße 14**
**40211 Düsseldorf (DE)**

# (54) PROCESS FOR THE RECOVERY OF EPSILON-CAPROLACTAM AND POLYETHER POLYURETHANE FROM NYLON 6 AND POLYETHER POLYURETHANE COMPRISING MATERIALS

(57) The present invention provides a process for recovering ε-caprolactam and polyether polyurethane from nylon 6 and polyether polyurethane comprising material in a plant, wherein the plant comprises a separation section [B], a depolymerization section [C], a recovery section [D], and a purification section [E]. The invention further provides apparatus suitable for carrying out the above process and polyether polyurethane and ε-caprolactam obtained by the process of the invention.

FIG. 1

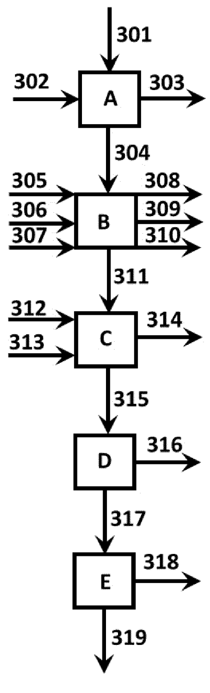


EP 4 306 584 A1

## Description

### TECHNICAL FIELD OF THE INVENTION

The present invention relates to a process for the recovery of ε-caprolactam from nylon 6 and polyether polyurethane comprising materials. More particularly, the present invention relates to a process for the recovery of both purified ε-caprolactam and polyether polyurethane from nylon 6 and polyether polyurethane comprising materials on an industrial scale. Further, the invention relates to a plant configured to carry out the process of the invention and to the recovered ε-caprolactam and polyether polyurethane obtainable by the process of the invention.

### BACKGROUND OF THE INVENTION

In 1938, Paul Schlack invented nylon 6 (CAS Number: 25038-54-4), also known as polyamide 6, PA6, N6, polycaprolactam, poly(hexano-6-lactam), poly(6-aminohexanoic acid), poly(hexamethylene adipamide) or poly[imino(1-oxohexane-1,6-diyl)].

Generally, nylon 6 is synthesized by ring-opening polymerization of ε-caprolactam at a temperature of about 260 °C in an inert atmosphere:

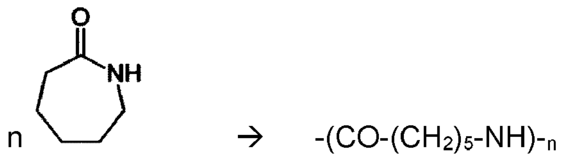


ε-caprolactam nylon 6

It is well-known that ε-caprolactam can be prepared by liquid phase Beckmann rearrangement of cyclohexanone oxime in the presence of oleum, a mixture of sulfuric acid and $SO_3$ or by gas phase Beckmann rearrangement of cyclohexanone oxime in the presence of a solid catalyst. Generally, this type of ε-caprolactam is referred to as "virgin ε -caprolactam". The required cyclohexanone oxime for the formation of (virgin) ε-caprolactam can be prepared from cyclohexanone that is mainly produced from benzene. Most of this benzene originates from non-renewable fossil resources like oil and coal.

Processes for the production of virgin ε-caprolactam are described in, e.g., Chapter "Caprolactam", in Ullmann's Encyclopedia of Industrial Chemistry (May 25, 2018), Wiley-VCH Verlag GmbH & Co. KGaA, Weinheim, Germany, electronically available via https://doi.org/10.1002/14356007.a05_031.pub3.

Processes for the production of nylon 6 are described in, e.g., Chapter "Polyamides", in Ullmann's Encyclopedia of Industrial Chemistry (January 15, 2013), Wiley-VCH Verlag GmbH & Co. KGaA, Weinheim, Germany, electronically available via https://doi.org/10.1002/14356007.a21_179.pub3.

Initially, pure nylon 6 was used for the production of textiles, including stockings. Later, in order to improve the properties of the fabrics, a wide array of blended fabrics has been developed and produced. Especially, polyether polyurethane has been incorporated into a wide range of nylon 6-based garments. A benefit of polyether polyurethane is its significant strength and elasticity and its ability to return to the original shape after stretching and faster drying than ordinary fabrics. Improved elasticity of fabric is often obtained by applying blended yarns that are made by blending nylon 6 fibers and polyurethane fibers. Examples of clothes containing fabric blends of nylon 6 and polyether polyurethane include stretchable stockings, underwear, and sportswear. Moisture permeability and waterproofness of fabrics can be obtained by processing (e.g., coating) the surface of nylon 6 fibers with a polyurethane resin. These surface treated yarns are often used for the manufacturing of, e.g., raincoats, cold weather clothes, and skiwear. Polyether polyurethanes that are used for the production of garments are also often called Spandex, Lycra or elastane.

Recycling of nylon 6 enables the conservation of fossil resources and can add value to the circular economy. Mechanical recycling of wasted nylon 6 includes processes in which wasted nylon 6 is transformed into secondary raw material or into products for which (preferably) the chemical structure of the material is only minimally changed. Conversion of nylon 6 and depolymerization of nylon 6 are two forms of chemical recycling of nylon 6. In the conversion recycling process nylon 6 is broken down into oil- or gas-like feedstock that can replace newly extracted fossil feedstock. The resulting products can be used to produce chemicals including monomer ε-caprolactam. In the depolymerization recycling process nylon 6 is broken down into its monomer building block ε-caprolactam. Depolymerization of nylon 6 into ε-caprolactam is the reverse reaction of the ring-opening polymerization of ε-caprolactam:

$-(CO-(CH_2)_5-NH)-_n$ → n 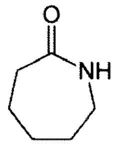

nylon 6 ε-caprolactam

Generally, the prior art chemical regeneration or recycling processes that depolymerize essentially pure nylon 6 into ε-caprolactam monomers comprise a hydrolytic degradation step at elevated temperature in the presence of water and a reclaiming step of the formed monomer by steam distillation.

Depolymerization recycling processes for nylon 6 are well developed for clean and rather pure wasted nylon 6 materials. Mechanical recycling always results in downcycling, so the properties of the products made of mechanically recycled nylon 6 are always worse than those made of virgin nylon 6. Chemical recycling of wasted nylon 6 containing materials, however, also may allow the production of high purity ε-caprolactam with properties similar to those of virgin made ε-caprolactam that afterwards can be converted into high grade nylon 6. Unfortunately, chemical recycling of wasted nylon 6 containing materials is often hampered by the presence of impurities in wasted nylon 6 materials. Generally, as a consequence of the presence of these impurities, the quality of the produced ε-caprolactam is inferior compared to virgin ε-caprolactam.

Depolymerization of blends of nylon 6 and polyether polyurethanes has many disadvantages compared to depolymerization of rather pure nylon 6. These disadvantages include amongst others blockage of piping and other pieces of equipment by polyether polyurethanes and their decomposition products, inferior quality of the produced ε-caprolactam due to the presence of decomposition products of polyether polyurethanes, (strongly) reduced ε-caprolactam recovery yields, poisoning of the depolymerization catalyst and thus higher consumption of depolymerization catalyst. Another disadvantage of the addition of blends of nylon 6 and polyether polyurethanes to a depolymerization reactor is that the polyether polyurethanes are destroyed. Thus, the recycling of the polyether polyurethanes, which are more valuable than nylon 6, is not possible.

Virgin elastane fibers are produced by dry or wet spinning processes that start from an elastane spinning solution that is made up of polyurethanes in suitable solvents such as dimethylacetamide or dimethylformamide.

Direct recycling of normal (pure) elastane wasted fibers, e.g., from yarn production, by dissolution in the employed spinning solvents proofed to be unsuccessful due to the high viscosities that prevented further processing.

US6830715B1 describes a method for producing elastane threads from spinning solutions using recycled elastane material that overcomes the before mentioned viscosity issue by addition of a secondary aliphatic amine to a mixture of (cut) elastane fibers and spinning solvent. The dissolving of the (cut) elastane fibers is realized at a temperature of 60 °C to 150 °C.

In the past already several attempts have been described for the recycling of blends of nylon 6 and polyether polyurethanes.

JP2011088943A describes a pre-treatment method for separating polyether polyurethane from a nylon 6 product containing polyether polyurethane that is followed by depolymerization of the residual nylon 6. The pre-treatment includes heating the nylon 6 product containing polyether polyurethane together with a cyclic amide containing solvent at temperatures from 80 °C to the boiling point of the solvent. JP2011088943A states that the amount of the cyclic amide compound in the cyclic amide compound solvent is preferably 50% by weight or more, more preferably 85% by mass or more. JP2011088943A states further that the cyclic amide compound solvent may contain components other than the cyclic amide compound such as water and an organic solvent, and water is particularly preferable from the viewpoint of operability. The experiments described in the patent were performed with aqueous solutions that contain 50 and 85 % by weight N-methylpyrrolidone, 85 % by weight 2-pyrrolidone or 85 % by weight 2-piperidin as solvent at a temperature of 110 °C during a period of 2 hours. As a result of this treatment, the polyether polyurethane is partly decomposed and dissolved in the solvent. Consequently, the resulting nylon 6 product from which polyether polyurethane has been removed and from which the solution is separated by filtration, is depolymerized. However, JP2011088943A is silent about recovery of the polyether polyurethane that is partly decomposed. Neither the fate of the used solvent is described.

WO2013032408A1 describes a method of recycling polyamide fibers including polyamide 6 and polyamide 6,6 comprising polyamide and spandex. Spandex fibers are removed from the elastomeric fabric by a process comprised of: controlled thermal degradation of spandex, controlled washing treatment for removal of spandex or its degradation products from high purity polyamide by using a suitable and sustainable solvent, preferably ethanol, and the final step to remove excess solvent from the polyamide fibers. The temperature range used during thermal treatment of polyamide fibers (including polyamide 6 fiber) is 150 °C - 220 °C, preferably from 190 °C - 216 °C and preferably during a period of 0.5 to 4 hours. Thereafter, the heat-treated fabric is washed with preferably ethanol to remove the spandex and its degraded components at temperatures ranging from 5 °C to 78 °C. WO2013032408A1 is silent about the recovery of

spandex and its degraded components from the washing solvent.

In view of the above, up to now no process exists for the recovery of both purified ε-caprolactam and polyether polyurethane from nylon 6 and polyether polyurethane comprising materials on an industrial scale. The reason why no recycling processes for the recovery of both purified ε-caprolactam and polyether polyurethane from nylon 6 and polyether polyurethane comprising materials on an industrial scale are realized lies in the fact that in all processes to bring the elastane into solution it is necessary to apply high solution temperatures, usually well above 100 °C. At these temperatures, however, degradation of elastane starts and only inferior quality material is obtained after recovery. As a consequence, the fate of the recovered elastane is often limited to incineration and land filling. Another disadvantage of dissolution processes at high temperatures is the formation of degradation products of the applied solvent, which may hinder the recovery of nylon 6 and polyether polyurethane and the recycling of the applied solvent.

Environmental concerns regarding the production and the usage of these nylon 6 and polyether polyurethane comprising materials relate to the in-process and the post-consumer generated wastes. These concerns could be mitigated by recycling individual components, such as nylon 6 and polyether polyurethane, from nylon 6 and polyether polyurethane comprising materials that are no longer used or disposed. As textile, nylon 6 and polyether polyurethane comprising materials are a particularly relevant source of waste. These often contain nylon 6 and polyether polyurethane in significant amounts. Thus, if a feasible process existed for recovering nylon 6 and polyether polyurethane from these composite waste materials, this would not only benefit the environment, but also present an economically valuable new source of nylon 6 and polyether polyurethane.

From the viewpoint of reducing carbon dioxide emission, it is of eminent importance to recycle nylon 6 and polyether polyurethane comprising materials and thereby recovering purified ε-caprolactam and polyether polyurethane.

And there is a need to provide high purity grade ε-caprolactam and polyether polyurethane from nylon 6 and polyether polyurethane comprising material that has significantly lower carbon footprint than the ε-caprolactam produced by a process using virgin ε-caprolactam obtained via new synthesis, e.g., by Beckmann rearrangement of cyclohexanone oxime, and polyether polyurethane produced by *de novo* synthesis of polyether polyurethane.

The raw material price of virgin polyether polyurethane is high, in general even more than two times higher than that of virgin nylon 6. Thus, there is a large economic incentive for the recovery and re-use (recycling) of both wasted polyether polyurethane and nylon 6.

A problem with the prior art processes is that they consume a large amount of energy for the recovery of dissolved elastane from solutions because the dissolved elastane is typically obtained by removing the solvent by evaporation.

And there is a need to purify the crude ε-caprolactam obtained by depolymerization of polyether polyurethane from nylon 6 and polyether polyurethane comprising materials without using oxidation agents like potassium permanganate ($KMnO_4$) or adsorbents like (activated) carbon and kieselguhr. Technologies that are based on these oxidation agents and adsorbents are quite laborious and produce solid wastes.

Another disadvantage of the prior art is that a suitable recovery and re-use (recycling) strategy of solvent(s) used for recovery and re-use (recycling) of polyether polyurethane and nylon 6 from nylon 6 and polyether polyurethane comprising materials is not provided or known.

Currently, no processes are available to recover high purity ε-caprolactam from nylon 6 and polyether polyurethane comprising materials despite the urgent need for such processes. In particular, there is an urgent need for high purity ε-caprolactam recovery processes that can replace virgin ε-caprolactam grades for high demanding applications, like for high speed melt spinning during textile fiber production.

Finally, there is a need for processes that allow the recovery of pure ε-caprolactam and polyether polyurethane from nylon 6 and polyether polyurethane comprising materials on an industrial scale in order to process the huge amounts of nylon 6 and polyether polyurethane comprising materials that are wasted annually.

## SUMMARY OF THE INVENTION

It is an object of the present invention, to satisfy one or more of the above-described needs and to overcome or alleviate the disadvantages associated with the prior art methods.

In particular, it is an object of the present invention to provide a process for recovering ε-caprolactam from a nylon 6 and polyether polyurethane comprising material. It is also an object of the present invention to provide a process for recovering both purified ε-caprolactam and polyether polyurethane from nylon 6 and polyether polyurethane comprising materials.

It is a further object of the invention to provide a process for recovery of both purified ε-caprolactam and polyether polyurethane from nylon 6 and polyether polyurethane comprising materials on an industrial scale.

It is also an object of the invention to provide a process for recovery of both purified ε-caprolactam and polyether polyurethane from nylon 6 and polyether polyurethane comprising materials in an economically responsible manner. In this regard, it is in particular an object of the present invention to provide a process that is suitable to recover both purified

ε-caprolactam and polyether polyurethane from nylon 6 and polyether polyurethane comprising materials and that does not exceed the production costs of virgin high purity ε-caprolactam and polyether polyurethane.

It is also an object of the invention to provide a process for purification of crude ε-caprolactam obtained by depolymerization of nylon 6 and polyether polyurethane comprising materials that does not produce solid wastes.

It is a further object of the invention to provide a process for recovery of both purified ε-caprolactam and polyether polyurethane from nylon 6 and polyether polyurethane comprising materials which is characterized by a significantly lower carbon footprint than a process for producing virgin polyether polyurethane, e.g., by reaction of polyether polyols and diisocyanate monomer, and ε-caprolactam via new synthesis, e.g., by Beckmann rearrangement of cyclohexanone oxime.

It is also an object of the present invention to provide a process for recovering high purity ε-caprolactam from nylon 6 and polyether polyurethane comprising materials that can replace high purity virgin ε-caprolactam for all applications including high speed melt spinning of nylon 6 for the production of thin textile fibers.

The invention therefore also aims to provide a process that reduces the environmental burden of wasted nylon 6 and polyether polyurethane comprising materials.

One or more further objects may become apparent from the remainder of the description.

All or at least some of the aforementioned objects are solved or at least mitigated to a large extent by the process of claim 1, the plant of claim 13 and the products of claims 14 and 15.

The present invention provides a process for recovering ε-caprolactam and polyether polyurethane from a nylon 6 and polyether polyurethane comprising material in a plant, wherein the plant comprises

- a separation section [B],
- a depolymerization section [C],
- a recovery section [D], and
- a purification section [E],

and wherein the process comprises the steps of:

a) charging the nylon 6 and polyether polyurethane comprising material to the separation section [B];

b) separating the nylon 6 and polyether polyurethane comprising material in a nylon 6 rich stream and a polyether polyurethane rich stream in the separation section [B] by selective dissolution of the polyether polyurethane in an organic solvent at a temperature below 100 °C, preferably at a temperature ranging from 0 °C to 100 °C, more preferably at a temperature ranging from 10 °C to 90 °C, even more preferably at a temperature ranging from 10 °C to 80 °C, and most preferably at a temperature ranging from 20 °C to 75 °C, wherein the polyether polyurethane rich stream is a solution comprising the organic solvent and polyether polyurethane;

c.1) discharging the nylon 6 rich stream from separation section [B] and charging the nylon 6 rich stream to depolymerization section [C] wherein the nylon 6 content of the nylon 6 rich stream is at least 85 % by weight on dry-weight basis;

c.2) depolymerizing the nylon 6 in the nylon 6 rich stream in the depolymerization section [C] at a temperature ranging from 180 °C to 400 °C, preferably from 200 °C to 350 °C, more preferably from 220 °C to 340 °C, and most preferably from 240 °C to 325 °C so that an ε-caprolactam comprising stream is obtained and discharging the obtained ε-caprolactam comprising stream from the depolymerization section [C];

c.3) recovering crude ε-caprolactam from said ε-caprolactam comprising stream in the recovery section [D];

c.4) purifying the crude ε-caprolactam obtained in the recovery section [D] in the purification section [E] to obtain purified ε-caprolactam wherein the purification comprises the steps of

(iv) obtaining purified ε-caprolactam by crystallization of ε-caprolactam from a solution comprising ε-caprolactam and impurities at a temperature of 10 °C to 95 °C.

d.1) recovering polyether polyurethane from the polyether polyurethane rich stream in the separation section [B]; and

d.2) discharging said recovered polyether polyurethane from the separation section [B] wherein the polyether polyurethane content of the discharged stream is at least 85 % by weight on dry-weight basis.

It was surprising to see that combining the special sequence of processing steps and process conditions according to the invention, i.e., the sequence of the above-defined separation, depolymerization, recovery and purification steps, allows to recover ε-caprolactam from nylon 6 and polyether polyurethane comprising materials in high yields and in a straight-forward and economically reasonable manner. The process of the invention is economically reasonable and advantageous from several points of view. Firstly, the process of the invention is suitable for a large variety of material derived from nylon 6 and polyether polyurethane comprising materials that can, e.g., differ in their overall composition and/or in their polyether polyurethane content and/or in their nylon 6 content. Secondly, the process of the invention allows to effectively separate nylon 6 from polyether polyurethane compounds so that high-grade nylon 6 can

be obtained. Thirdly, the process of the invention is so effective that ε-caprolactam can be obtained in high yields. Fourthly, the process of the invention allows the recovery of polyether polyurethane that can be re-used to replace polyether polyurethane produced by *de novo* synthesis, e.g., by reaction of polyether polyols and diisocyanate monomer. Fifthly, the process of the invention allows to effectively separate ε-caprolactam from non-ε-caprolactam compounds so that high purity grade ε-caprolactam can be obtained that can replace high purity virgin ε-caprolactam for all applications including high speed melt spinning of nylon 6 for the production of thin textile fibers. Lastly, the process of the invention allows to produce ε-caprolactam with a significantly lower carbon footprint compared to ε-caprolactam produced by *de novo* synthesis of ε-caprolactam, e.g., by the Beckmann rearrangement of cyclohexanone oxime. The process of the invention allows to process nylon 6 and polyether polyurethane comprising materials efficiently and to reduce the environmental burden of said products. In particular, the process of the invention allows the production of polyether polyurethane with a carbon footprint of less than 1 kg $CO_2$ per kg polyether polyurethane, which is a substantial improvement compared to the 4.8 kg $CO_2$ per kg polyether polyurethane associated with the production of "virgin" polyether polyurethane obtained by chemical synthesis. In particular, the process of the invention allows the production of purified ε-caprolactam with a carbon footprint of less than 3 kg $CO_2$ per kg purified ε-caprolactam, which is a substantial improvement compared to the 6.4 to 7.5 kg $CO_2$ per kg ε-caprolactam associated with the production of "virgin" ε-caprolactam obtained from a Beckmann rearrangement of cyclohexanone oxime.

Next to the process of the invention, the present invention also provides a plant for the production of purified ε-caprolactam and polyether polyurethane from a nylon 6 and polyether polyurethane comprising material, wherein the plant comprises

- optionally, a pre-treatment section [A],
- a separation section [B],
- a nylon 6 depolymerization section [C],
- an ε-caprolactam recovery section [D],
- an ε-caprolactam purification section [E], and

wherein the chemical plant is configured for carrying out a process of the invention.

The present invention also provides purified ε-caprolactam obtained via separation from nylon 6 and polyether polyurethane comprising material according to a process of the invention, wherein the ε-caprolactam has a product carbon footprint of less than 3.0 kg $CO_2$ eq. per kg purified ε-caprolactam.

The present invention also provides polyether polyurethane obtained via separation from nylon 6 and polyether polyurethane comprising material according to a process of the invention, wherein the polyether polyurethane has a product carbon footprint of less than 1.0 kg $CO_2$ eq. per kg recovered polyether polyurethane.

Advantageous embodiments of the invention are indicated in the dependent claims and are explained in more detail in the following.

## DETAILED DESCRIPTION OF THE INVENTION

### The nylon 6 and polyether polyurethane comprising material

The process of the invention uses nylon 6 and polyether polyurethane comprising material as starting material. The nylon 6 and polyether polyurethane comprising material can be (used) products comprising nylon 6 and polyether polyurethane or materials derived therefrom. The nylon 6 and polyether polyurethane comprising material can be nylon 6 and polyether polyurethane comprising materials, i.e. a plurality of nylon 6 and polyether polyurethane comprising materials or mixtures thereof. Typically, nylon 6 and polyether polyurethane comprising materials are solid materials, in particular fibers, yarns (i.e., strands of fibers that are spun together) or fiber-based fabrics, whereby the fibers are a blend of nylon 6 fibers and polyurethane fibers, or whereby the fibers are obtained by coating the surface of nylon 6 fibers with a polyether polyurethane resin.

The nylon 6 and polyether polyurethane comprising material can be both of pre-consumer and post-consumer origin. The nylon 6 and polyether polyurethane comprising material can be a mixture of different nylon 6 and polyether polyurethane comprising materials or a mixture of one or more nylon 6 and polyether polyurethane comprising materials with one or more different materials.

Nylon 6 and polyether polyurethane comprising materials can contain additional components. Such components can be added, e.g., during the polymerization, the formation of the fibers or afterwards to achieve a desired property variation. These compounds include, e.g., brightening agents, stiffening agents, anti-static lubricants, colorants, brightening agents, spin finish, agents for surface smoothness, anti-oxidation agents, UV stabilizers, and so on. The quantities of these additional components are dependent on the application of the nylon 6 and polyether polyurethane comprising materials.

The weight to weight ratio of nylon 6 to polyether polyurethane in the nylon 6 and polyether polyurethane comprising material can vary. Preferably, the weight to weight ratio of nylon 6 to polyether polyurethane ranges from ca. 1 : 1 to ca. 100 : 1, most preferably from ca. 3 : 1 to ca. 20 : 1.

Polyurethanes are produced by reacting an isocyanate containing two or more isocyanate groups per molecule with a polyol containing on average two or more hydroxyl groups per molecule in the presence of a catalyst or by activation with ultraviolet light. The main ingredients to make a polyurethane are di- and tri-isocyanates and polyols. Diisocyanate monomer methylene diphenyl diisocyanate (MDI) is the most commonly used isocyanate for the production of polyurethanes for garment applications.

Polyether polyurethanes are made by reaction of isocyanates with polyether polyols. Polyether polyols are generally made by polymerizing a cyclic ether compound onto an initiator compound. The cyclic ethers most commonly used in polyether polyol production are ethylene oxide, propylene oxide and 1,4-butylene oxide or tetrahydrofuran (to produce poly(butylene oxide) polyols). Poly(butylene oxide) polyols are used mostly in applications in which highly hydrophobic characteristics are needed.

The use of the term "ca." or "about" in connection with numerical values herein indicates that the numerical values may be affected by measurement errors which typically change the numerical values by not more than $\pm$ 5%. Numerical values disclosed herein together with the term "ca." or "about" are also meant to be disclosed as such, i.e., without the term "about". Further, numerical ranges as stated herein are meant to include and disclose each and every value within that range. All upper and lower end points of ranges for the same parameter disclosed herein are combinable with each other. All ranges for different parameters disclosed herein are combinable with each other. In particular, the ranges of different or the same "preference level" are particularly compatible with each other. As used in the present disclosure and claims, the singular forms "a," "an," and "the" include plural forms, especially in the sense of "one or more", unless the context clearly dictates otherwise.

Possible pre-treatment steps

Before being subjected to step a) of the process of the invention, the nylon 6 and polyether polyurethane comprising material can undergo a pre-treatment in pre-treatment section [A], in particular size reduction in a mechanical size reduction section [A] and/or a cleaning in a cleaning section [ω]. The pre-treatment can be performed at a different site than where the separation section [B] is located. However, preferably, the process of the invention is performed in a plant that further comprises a pre-treatment section [A], and wherein prior to step a) the nylon 6 and polyether polyurethane comprising material is subjected to a pre-treatment in a pre-treatment section [A], in particular to a cleaning in a cleaning section [ω] and/or to a mechanical size reduction in a mechanical size reduction section [λ]. This has the advantage that the nylon 6 and polyether polyurethane comprising material that is charged to the separation section [B] are less contaminated with foreign materials, which improves the yield and the purity of the ε-caprolactam and the polyether polyurethane that are produced in the plant of the invention configured to carry out the process of the invention. Another advantage is that nylon 6 and polyether polyurethane comprising material that is size reduced can be more easily handled.

The nylon 6 and polyether polyurethane comprising material preferably is or is derived from a used or wasted nylon 6 and polyether polyurethane comprising material. The dimensions and shapes of the material depends very much on the exact application that it is derived from. Nylon 6 and polyether polyurethane comprising materials that can be used according to the invention range from separate fibers and yarns, optionally presented in bobbins to woven fabrics and garments. Preferably, the nylon 6 and polyether polyurethane comprising material is a wasted or used nylon 6 and polyether polyurethane comprising material.

Especially wasted or used nylon 6 and polyether polyurethane comprising materials can be contaminated with various types of dirt (e.g., with mud, oil, paint or grease).

(Wasted) nylon 6 and polyether polyurethane comprising materials can be mixed with a whole range of other materials, such as rocks, glass, metal materials, organic waste and other polymer litter (e.g., polyamide 6,6, polyethylene terephthalate (PET), polypropylene (PP) or polyethylene (PE)).

Preferably, the nylon 6 and polyether polyurethane comprising material is fragmented into pieces before they are separated in the separation section [B] in step b). This mechanical pre-treatment, i.e., the mechanical comminution or fragmentation of the nylon 6 and polyether polyurethane comprising material, can be achieved, e.g., by cutting, punching, shredding, milling, grinding and/or chipping. In a preferred embodiment, the nylon 6 and polyether polyurethane comprising material is charged into the separation section [B] of step a) in the form of fragmented pieces. Using fragmented pieces has the advantage that the pieces can be more easily handled and/or cleaned by washing with a solvent. In a preferred embodiment, the pieces have on average, along the longest axis of the piece, a length of 1 mm to 100 m, preferably from 3 mm to 1 m and most preferably from 1 cm to 50 cm. The skilled person can easily determine the average length along the longest axis of the employed pieces by first taking a representative sample of the pieces, then measuring the length of the longest axis of each of these pieces (e.g., 50 pieces), and finally calculating the average

value of all these individual measurements. The preferred particle dimensions can also be described in terms of average particle weight. Preferably, the pieces of nylon 6 and polyether polyurethane comprising material have an average particle weight from 0.01 gram to 25 kg, preferably from 0.05 gram to 1 kg and most preferably from 0.1 gram to 100 gram. Experiments have shown that pieces of nylon 6 and polyether polyurethane comprising material of the aforementioned size or weight dimension are particularly well suited for handling in the process of the invention and/or cleaning by washing with a solvent.

Optionally, prior to the mechanical comminution or fragmentation of the nylon 6 and polyether polyurethane comprising material, large metal fragments, rocks and other disturbing materials that cause severe wear of the equipment used for the mechanical comminution or fragmentation are removed. Preferably, also foreign materials including, but not limited to, polyethylene, polypropylene and polyamide 6,6 comprising materials are removed prior to the mechanical comminution or fragmentation of the nylon 6 and polyether polyurethane comprising materials. The removal of foreign materials can be done mechanically or manually. The removal of these disturbing materials has the advantage that the maintenance cost of the equipment used for the mechanical comminution or fragmentation can be reduced to a large extent. In addition, the nylon 6 and polyether polyurethane content of the material obtained after mechanical comminution or fragmentation is higher than without removal of the disturbing materials. In particular the removal of polyamide 6,6 is advantageous because it disturbs the depolymerization of nylon 6, reduces the recovery yield of ε-caprolactam and disturbs the subsequent purification of the recovered ε-caprolactam. The term "foreign material" as used herein means a non-nylon 6 and non-polyether polyurethane material or compound.

Optionally, foreign materials are separated from the nylon 6 and polyether polyurethane comprising material that has been mechanically comminuted or fragmented. To this end, various separation processes are applicable, including, but not limited to, density separation and magnetic separation. In density separation, materials of different densities are placed in a liquid of intermediate density, where the less dense material floats and separates out from the more dense sinking material. In practice, density separation is often done by a series of density separation stages. E.g., in one stage, the high density materials like rocks, sand and metals (including iron and lead) are separated off, while in another stage low density materials, like polyolefins polypropylene and polyethylene, are separated off. Magnetic separation is the process of separating components of mixtures by using magnets to attract magnetic materials. The process that is preferably used for magnetic separation detaches non-magnetic material from magnetic material. The removal of foreign materials in the comminuted or fragmented nylon 6 and polyether polyurethane comprising material is advantageous because such materials can disturb the separation of nylon 6 and polyether polyurethane, the depolymerization of nylon 6, reduce the recovery yield of ε-caprolactam and/or disturb the subsequent purification of the recovered ε-caprolactam.

Optionally, in particular in the pre-treatment step described above, the nylon 6 and polyether polyurethane comprising material is cleaned by washing with a solvent, preferably at least with water, prior to being charged to the separation section [B]. Solvent as used herein can be a single solvent or a mixture of different solvents. Preferably, washing agents ranging in concentrations from 0 to 20 % by weight relative to the solvent are added to the solvent for an improved washing efficiency. NaOH is a preferred washing agent. Even more preferably, an aqueous solution that contains 0 to 10 % by weight NaOH is used in the washing step, still more preferably 0 to 5 % by weight NaOH. The enhanced washing effect of NaOH is most probably caused by enhanced hydrolysis of molecules, including biopolymers and non-biopolymers. Preferably, the washing solvent is heated to further enhance the washing process. In another preferred embodiment, the washing process includes a final rinsing step with (clean) washing solvent without washing agent in order to remove residues of washing agent and dirt present that are adhering to the nylon 6 and polyether polyurethane comprising material.

The washing is preferably carried out under friction. Different types of industrial friction-applying washing systems are available on the market, like rotary plastic washers and (high speed) friction washers.

Washing of the nylon 6 and polyether polyurethane comprising material, in particular of mechanically comminuted or fragmented nylon 6 and polyether polyurethane comprising material, is advantageous because any (adhering) dirt is removed which consequently does not perturb the next steps of the process of the invention.

Optionally, the nylon 6 and polyether polyurethane comprising material is dried after the cleaning step and prior to being charged to the separation section [B]. This has the advantage that the weight of the cleaned nylon 6 and polyether polyurethane comprising material is reduced and that the next step is not affected by dilution or by contamination with the washing solvent.

The site where the pre-treatment of the nylon 6 and polyether polyurethane comprising material is performed and the site where the separation section [B] is located can be the same. However, preferably, one or more of the pre-treatment steps are done at a location different from the location of the separation section [B], e.g., near a plant where the (used or wasted) nylon 6 and polyether polyurethane comprising material is collected and/or at a location that is specialized in pre-treatment of nylon 6 and polyether polyurethane comprising material. A nylon 6 rich stream can then be obtained in the separation section [B] from nylon 6 and polyether polyurethane comprising material that has already been pre-treated.

The site where the separation of the nylon 6 and polyether polyurethane comprising materials into a nylon 6 rich stream and a polyether polyurethane rich stream is performed and the site where the depolymerization section [C] is located can be the same. However, preferably, separating the nylon 6 and polyether polyurethane comprising materials in a nylon 6 rich stream and a polyether polyurethane rich stream is done at a location different from the location of the depolymerization section [C], e.g., near a plant where virgin polyether polyurethane is produced and/or at a location that is specialized in handling and/or distilling organic solvents.

The site where the pre-treatment of the nylon 6 and polyether polyurethane comprising materials is performed and the site where the depolymerization section [C] is located can be the same. However, preferably, one or more of the pre-treatment steps are done at a location different from the location of the depolymerization section [C], e.g., near a plant where the wasted nylon 6 and polyether polyurethane comprising material is collected and/or at a location that is specialized in pre-treatment of nylon 6 and polyether polyurethane comprising materials.

## The charging step a)

In step a) of the process of the invention, the nylon 6 and polyether polyurethane comprising material that has been optionally pre-treated in pre-treatment section [A], is charged to the separation section [B].

In one embodiment, the nylon 6 and polyether polyurethane comprising material is mechanically compressed into a smaller volume prior to being charged to the separation section [B]. This has the advantage that less volume is needed for intermediate storage and transport and can also facilitate dosing to the separation section [B].

In another preferred embodiment, the nylon 6 and polyether polyurethane comprising material is dried before being charged to the separation section [B], in particular after subjecting the nylon 6 and polyether polyurethane material to a cleaning step. This has the advantage that less or no solvent is introduced with the material into the separation section [B]. Solvent, especially water, introduced in the separation section [B] may negatively influence the separation process (e.g., reduced dissolution rates of polyether polyurethane from the nylon 6 and polyether polyurethane comprising material in an organic solvent are obtained).

The nylon 6 and polyether polyurethane comprising material is preferably fed to the dissolution section [$\alpha$], in particular the corresponding dissolution vessel(s) contained therein, as a solid phase.

The feeding of the nylon 6 and polyether polyurethane comprising material to the separation section [B] (e.g., feeding to the polyether polyurethane dissolution section) can be realized by continuous or by intermittent dosing of the nylon 6 and polyether polyurethane comprising material.

## The separation step b)

In the separation section [B], the nylon 6 and polyether polyurethane comprising material is separated to form a nylon 6 rich stream and a polyether polyurethane rich stream.

Preferably, the process of the invention employs a separation section [B] comprising a dissolution section [$\alpha$], a washing section [$\beta$], a precipitation section [$\gamma$], and a solvent distillation section [$\delta$], and comprises the following steps in the separation section:

b.1) charging an organic solvent, and the nylon 6 and polyether polyurethane comprising material to the dissolution section [$\alpha$];

b.2) dissolving polyether polyurethane from the nylon 6 and polyether polyurethane comprising material in an organic solvent in the dissolution section [$\alpha$], so that a polyether polyurethane rich stream comprising organic solvent and dissolved polyether polyurethane, and a stream comprising non-dissolved nylon 6 are obtained and discharging the obtained streams from the dissolution section [$\alpha$];

b.3) charging a second solvent and said polyether polyurethane rich stream comprising organic solvent and dissolved polyether polyurethane to the precipitation section [$\gamma$] so that polyether polyurethane is precipitated from a mixture comprising organic solvent and second solvent;

b.4) recovering said precipitated polyether polyurethane from said mixture comprising organic solvent and second solvent and discharging said precipitated polyether polyurethane from the precipitation section [$\gamma$];

b.5) discharging the mixture comprising organic solvent and second solvent from which precipitated polyether polyurethane has been recovered in step b.4) from the precipitation section [$\gamma$] and charging said mixture to the solvent distillation section [$\delta$];

b.6) charging a third solvent and the stream comprising non-dissolved nylon 6 to the washing section [$\beta$];

b.7) washing the stream comprising non-dissolved nylon 6 with the third solvent in the washing section [$\beta$], so that a nylon 6 rich stream and a mixture comprising organic solvent and third solvent are obtained;

b.8) discharging said nylon 6 rich stream from the washing section [$\beta$];

b.9) discharging the mixture comprising organic solvent and third solvent that is obtained in step b.7) from the

washing section [β] and charging said mixture partly or completely to the solvent distillation section [δ];
b.10) separating organic solvent from the second solvent and the third solvent by distillation in the solvent distillation section [δ] and discharging the second solvent, the third solvent, and the separated organic solvent from the solvent distillation section [δ].

Optionally the second solvent (see step b.3)) can be partly or completely the mixture comprising organic solvent and third solvent that is obtained in step b.7) from the washing section [β].

Preferably, the separation section [B] employed in the process of the invention comprises a dissolution section [α], a washing section [β], a precipitation section [γ], and a solvent distillation section [δ]. Such a separation section [B] is particularly suitable to perform the separation of the nylon 6 and polyether polyurethane comprising material into a nylon 6 rich stream and a polyether polyurethane rich stream according to the process of the invention. Surprisingly, the separation of the nylon 6 and polyether polyurethane comprising material into a nylon 6 rich stream and a polyether polyurethane rich stream is particularly effective when performing the process steps b.1) to b.10) in the separation section [B] comprising a dissolution section [α], a washing section [β], a precipitation section [γ], and a solvent distillation section [δ]. Steps b.1) to b.10) taking place in the preferred separation section [B] are further explained in the following. Steps b.1) to b.10) can replace process steps a) and b) in the process of the invention. Step b.1) thereby corresponds to step a).

A "rich stream" as used herein is a stream which contains the enriched component in a greater amount than another stream obtained in the same process step. The nylon 6 rich stream therefore contains more nylon 6 than the polyether polyurethane rich stream. In contrast, the polyether polyurethane rich stream contains more polyether polyurethane than the nylon 6 rich stream.

In particular, the nylon 6 rich stream has a weight to weight ratio of nylon 6 to polyether polyurethane that is higher than the weight to weight ratio of nylon 6 to polyether polyurethane of the nylon 6 and polyether polyurethane comprising material.

Preferably, the nylon 6 rich stream has a weight to weight ratio of polyether polyurethane to nylon 6 that is at least two times lower than the weight to weight ratio of polyether polyurethane to nylon 6 of the nylon 6 and polyether polyurethane comprising material, more preferably at least three times lower.

The polyether polyurethane rich stream has a weight to weight ratio of polyether polyurethane to nylon 6 that is higher than the weight to weight ratio of polyether polyurethane to nylon 6 of the nylon 6 and polyether polyurethane comprising material.

Preferably, the polyether polyurethane rich stream has a weight to weight ratio of polyether polyurethane to nylon 6 that is at least two times higher than the weight to weight ratio of polyether polyurethane to nylon 6 of the nylon 6 and polyether polyurethane comprising material, more preferably at least three times higher.

<u>The charging to separation section step (step b.1)):</u>

In separation section [B], an organic solvent, and the nylon 6 and polyether polyurethane comprising material are charged to the dissolution section [α].

The charging of the nylon 6 and polyether polyurethane comprising material to the dissolution section [α] is the charging of the nylon 6 and polyether polyurethane comprising material that are optionally pre-treated in pre-treatment section [A] as mentioned in step a) of the invention.

The organic solvent that is charged to the dissolution section [α] can be any organic solvent in which polyether polyurethane can be dissolved, in particular at temperatures below 100 °C, and in particular in less than 24 or less than 6 hours. Organic solvent as used herein can also mean a mixture of organic solvents or a liquid composition comprising more than 60 vol.-%, preferably more than 70 vol.-%, 80 vol.-%, or 90 vol.-% organic solvent, wherein the mixture is able to dissolve polyether polyurethane at temperatures below 100 °C, preferably at a temperature ranging from 0 °C to 100 °C, more preferably at a temperature ranging from 10 °C to 90 °C, even more preferably at a temperature ranging from 10 °C to 80 °C, and most preferably at a temperature ranging from 20 °C to 75 °C. Preferably, the organic solvent combines a good, in particular very good, solubility for polyether polyurethane and a poor, in particular very poor, solubility for nylon 6. These properties can be tested in simple dissolution tests with nylon 6 and polyether polyurethane comprising material or mixtures of nylon 6 and polyether polyurethane. According to a preferred embodiment, the organic solvent comprises or is selected from N,N-dimethylformamide (DMF, $(CH_3)_2NC(=O)H$), dimethylacetamide (DMAc, $CH_3C(=O)N(CH_3)_2$), 1,4-dioxane (dioxane, $C_4H_8O_2$), N-alkyl-2-pyrrolidones such as N-methyl-2-pyrrolidone (NMP, $C_5H_9NO$), tetrahydrofuran (THF; a.k.a. oxolane, $C_4H_8O$) and combinations thereof. Even more preferably, the organic solvent is selected from N,N-dimethylformamide and dimethylacetamide. Most preferably, the organic solvent is dimethylacetamide.

The organic solvent that is charged to the dissolution section [α] can be a separated organic solvent (i.e., recycled organic solvent or an organic solvent from inside the process, that has been recovered in a solvent distillation section), fresh organic solvent (i.e., solvent from outside the process) or a combination of separated organic solvent and

fresh organic solvent.

The organic solvent that is charged to the dissolution section [α] preferably has a low water content. Even more preferably, the organic solvent is dried (preferably by distillation or applying a drying agent, like an inorganic salt such as $Na_2SO_4$, a zeolite, silica and alumina) before being charged to the dissolution section [α]. Drying needs not necessarily be performed to completion. Typically, less than 1 wt.%, preferably, less than 0.1 wt.%, more preferably, less than 0.02 wt.% water remaining after drying the organic solvent is acceptable.

The dissolving step (step b.2)):

In dissolution section [α] polyether polyurethane is dissolved from the nylon 6 and polyether polyurethane comprising material in the organic solvent that was charged to the dissolution section [α] in step b.1). Hereby a polyether polyurethane rich stream comprising organic solvent and dissolved polyether polyurethane, and a stream comprising non-dissolved nylon 6 are obtained that are discharged from dissolution section [α].

Preferably, dissolution of polyether polyurethane will be complete in 0.1 hour to 24 hours, more preferably in 0.5 hour to 6 hours. Complete means that the dissolution has reached a plateau with no substantial further dissolution occurring at later timepoints.

The temperature in the dissolution section [α] can vary and is preferably below 120 °C, more preferably below 110 °C, even more preferably below 100 °C and most preferably below 80 °C. Preferably, the temperature in the dissolution section [α] may range from 0 °C to 100 °C, more preferably from 10 °C to 90 °C, even more preferably from 10 °C to 80 °C, and most preferably from 20 °C to 75 °C. Experiments have shown that these temperature ranges result in a particularly complete dissolution of polyether polyurethane while maintaining its integrity in organic solvents. This has the advantage that the polyether polyurethane present in the nylon 6 and polyether polyurethane comprising material can be dissolved while maintaining its integrity.

The amount of solvent (expressed in weight, in particular in tons) in the dissolution section [α] can vary and may range from 0.5 to 100 times, preferably from 1 to 60 times, more preferably from 2 to 20 times, most preferably from 3 to 10 times the amount of polyether polyurethane (expressed in weight, in particular in tons) in the dissolution section [α]. A low amount of solvent reduces the dissolution rate of polyether polyurethane and may result in a too low or incomplete dissolution of the polyether polyurethane. A large amount of solvent gives a high dissolution rate of polyether polyurethane, however requires more energy and costs for the recovery of the used organic solvent. The skilled person is able to determine the optimal ratio of solvent to polyether polyurethane or nylon 6 and polyether polyurethane comprising material by way of routine tests.

The dissolution section [α] may comprise one or more dissolution vessels that are operated in series or in parallel. Preferably, these vessels are equipped with additional equipment that enhances friction, such as stirrers, mixers or agitators, which reduces the time of dissolution.

The precipitation step (step b.3)):

The polyether polyurethane rich stream comprising organic solvent and dissolved polyether polyurethane that was discharged from the dissolving section [α] and a second solvent are charged to the precipitation section [γ] where they mix so that polyether polyurethane is precipitated from a mixture comprising organic solvent and second solvent.

Preferably, the second solvent is a non-solvent (a.k.a. anti-solvent) for polyether polyurethane, i.e. a liquid in which polyether polyurethane is not soluble and that causes polyether polyurethane to precipitate. The addition of this second solvent reduces the solvent power for the polyether polyurethane dissolved in the solution, which leads to precipitation of particles of precipitated polyether polyurethane. The second solvent can be any solvent. Preferably, the second solvent is an aqueous solution or water, which has the advantage that it is an environmentally benign solvent.

In a preferred embodiment, the second solvent consists of part of or is the mixture comprising organic solvent and third solvent that is obtained in step b.7) from the washing section [β]. "Part of the mixture or is" as used herein is defined as any fraction between 1 and 100 wt.%, preferably, between 10 and 100 wt.%, more preferably, between 25 and 100 wt.%, even more preferably, between 75 to 100 wt.%. This has the advantage that less fresh solvent is needed for the precipitation of polyether polyurethane. Another advantage is that less solvent needs to be distilled in solvent distillation section [δ], which results in lower energy costs, smaller sized equipment, reduced investment costs and a more environmentally benign process.

The temperature in the precipitation section [γ] can vary and may range from 0 °C to 150 °C, preferably from 10 °C to 100 °C, more preferably from 15 °C to 80 °C and most preferably from 20 °C to 60 °C. Experiments have shown that these temperature ranges result in particularly complete precipitation of the dissolved polyether polyurethane.

The amount of second solvent charged to the precipitation section [γ] can vary and may range from 0.1 wt.% to 500 wt.%, preferably from 0.2 wt.% to 100 wt.%, more preferably from 0.5 wt.% to 50 wt.% and most preferably from 1 wt.% to 25 wt.% compared to the organic solvent charged to the precipitation section [γ].

A low amount of second solvent reduces the degree of precipitation of polyether polyurethane. A large amount of second solvent complicates the recovery of precipitated polyether polyurethane and the organic solvent. A large amount of second solvent also requires more energy and costs for the recovery of precipitated polyether polyurethane and the organic solvent. The skilled person will be able to find the optimal ratio of second solvent to polyether polyurethane rich stream for the specific solvent and equipment used by carrying out simple precipitation test trials.

The polyether polyurethane recovery step (step b.4):

In the polyether polyurethane recovery step precipitated polyether polyurethane is recovered from the mixture comprising organic solvent and second solvent and discharged from precipitation section [γ]. There are several suitable methods to recover the polyether polyurethane precipitate, including, but not limited to, filtration, centrifugation and decantation. Preferably, filtration is used to recover polyether polyurethane. For filtration, the solution containing the precipitate is charged to a filter, whereby the precipitate is expected to remain on the filter, while the liquid passes through it. When using centrifugation as recovery method, the solution containing the precipitate is rapidly rotated so that the solid precipitate settles (assuming that the solid precipitate has a higher density than the liquid). The compacted precipitate can also be obtained by pouring off the liquid. In decantation, the liquid layer is poured or suctioned away from the precipitate.

Optionally, the recovered precipitate is washed with a solvent before being discharged from precipitation section [γ]. Preferably, the solvent that is used to wash the recovered precipitate is either the organic solvent of step b.1) or second solvent.

Optionally, the recovered precipitate that is optionally washed with a solvent is dried before being discharged from precipitation section [γ]. It has been advantageously found that the precipitated and recovered polyether polyurethane that is discharged from the precipitation section [γ] in step b.4) of the method of the invention is of high quality and can be re-used, optionally in combination with fresh polyether polyurethane, in the production of textiles, which is a preferred embodiment of the invention.

The discharging solvent mixture step (step b.5)):

The mixture comprising organic solvent and second solvent from which precipitated polyether polyurethane has been recovered in step b.4) is discharged from precipitation section [γ] and charged to solvent distillation section [δ]. Optionally, solvent resulting from washing of the recovered precipitate is also discharged from precipitation section [γ] and charged to solvent distillation section [δ].

The charging to washing step (step b.6)):

In separation section [B], a third liquid solvent, and the stream comprising non-dissolved nylon 6 are charged to the washing section [β]. The stream comprising non-dissolved nylon 6 that is charged to the washing section [β] can comprise next to non-dissolved nylon 6 also organic solvent. The purpose of the washing section [β] is to recover organic solvent that is present in the stream comprising non-dissolved nylon 6, whereby a nylon 6 rich stream is obtained that contains less organic solvent than the stream comprising non-dissolved nylon 6.

Preferably, the third solvent has a good solubility for the organic solvent and a poor solubility for nylon 6. The third solvent can be any solvent. Preferably, the second solvent is water or an aqueous solution. Water as third solvent has the advantage that it is an environmentally benign solvent. The third and second solvent can be of the same composition, which has the advantage of simplifying the process by reducing the amount of different components needed.

The amount of third solvent charged to the washing section [β] can vary and may range from 10 wt.% to 1000 wt.%, preferably from 20 wt.% to 500 wt.%, more preferably from 50 wt.% to 400 wt.% and most preferably from 100 wt.% to 250 wt.% compared to the stream comprising non-dissolved nylon 6 that is charged to the washing section [β].

The washing step (step b.7)):

In washing section [β], the stream comprising non-dissolved nylon 6 is washed with the third solvent so that a nylon 6 rich stream and a mixture comprising organic solvent and third solvent are obtained.

The temperature in the washing section [β] can vary and may range from 0 °C to 150 °C, preferably from 10 °C to 100 °C, more preferably from 15 °C to 80 °C and most preferably from 20 °C to 60 °C. Experiments have shown that these temperature ranges resulted in a particularly complete recovery of the organic solvent from the stream comprising non-dissolved nylon 6.

The skilled person can determine by routine experimentation the amount of third solvent and the optimal temperature necessary for efficient washing of the stream comprising non-dissolved nylon 6.

The washing of the stream comprising non-dissolved nylon 6 with a third solvent can be performed in various devices, all of which are known to the skilled person.

The discharging of nylon 6 rich stream step (step b.8)):

From the washing section [β] the nylon 6 rich stream is discharged. This nylon 6 rich stream comprises third solvent and optionally organic solvent. Preferably, the organic solvent content of the nylon 6 rich stream is less than 25 wt.%, preferably less than 10 wt.%, more preferably less than 2 wt.% and most preferably less than 0.2 wt.% relative to the total weight of the nylon 6 rich stream. Optionally, the nylon 6 rich stream is dried prior to being discharged.

Typically, the obtained nylon 6 rich stream comprises a solid nylon 6 rich material. The term "solid" in this respect refers to the state of the material at room temperature (20 °C). At higher temperatures, the nylon 6 rich material can also be present as a melt. The nylon 6 rich material is stable and can be stored before being charged to depolymerization section in step c.1) of the process of the invention or transported to a depolymerization section at a different location for this purpose.

Preferably, the nylon 6 content of the resulting nylon 6 rich stream from washing section [β] is at least 85 %, more preferably at least 90 % and most preferably at least 95 % by weight on dry-weight basis.

Preferably, the polyamide 6 content of the resulting polyamide 6 rich stream from separation section [B] is at least 85 %, more preferably at least 90 %, even more preferably at least 95 % and most preferably at least 98 % by weight on dry-weight basis.

Determination of the nylon 6 content is a routine activity and can be performed by various methods, all of which are known to the skilled person. Preferably, the polyamide 6 content of the nylon 6 rich stream is determined by Thermogravimetric analysis (TGA) and/or Differential Scanning Calorimetry (DSC) (e.g., by method ISO 11357-3).

As used herein, the expression "dry weight basis" refers to the content of an ingredient expressed as a percentage of the total dry weight of the composition, wherein "dry weight" is the weight of a substance after removing the water and/or other liquids from the substance by drying until weight constancy.

The discharging of mixture comprising organic solvent and third solvent step (step b.9)):

From the washing section [β] the mixture comprising organic solvent and third solvent is discharged. Generally, the organic solvent and/or the third solvent are valuable compounds and recovery and reuse has great economic and environmental advantages. In a preferred embodiment of the invention, the mixture comprising organic solvent and third solvent is therefore charged to solvent distillation section [δ]. Optionally, the mixture comprising organic solvent and third solvent is first charged to and used in another section, in particular as second solvent in the precipitation section, before being charged to solvent distillation section [δ]. In a preferred embodiment, the mixture comprising organic solvent and third solvent is first partly or completely charged to the precipitation section [γ] and used as second solvent. This presents a particularly resourceful use and reuse of the solvents in the method of the invention.

The solvent separation step (step b.10)):

In solvent distillation section [δ], the different solvents used in the process of the invention are separated and recovered again. This allows them to be advantageously reused. In particular, the mixture comprising organic solvent and second solvent from which precipitated polyether polyurethane has been recovered and the mixture comprising organic solvent and third solvent that was discharged from washing section [β] are separated by distillation in the solvent distillation section [δ]. Hereafter, the mixture comprising organic solvent and second solvent from which precipitated polyether polyurethane has been recovered and the mixture comprising organic solvent and third solvent that was discharged from washing section [β] are called combined feed to solvent distillation section [δ].

From the solvent distillation section [δ] the separated organic solvent, the second solvent and the third solvent, and residues are discharged. In a preferred embodiment, the separated organic solvent that is discharged from the solvent distillation section [δ] in step b.10) is charged to the dissolution section [α] in step b.1). In another preferred embodiment, the second solvent and the third solvent that are discharged from the solvent distillation section [δ], optionally after separation from each other, are re-used in the precipitation section [γ] and/or in the washing section [β].

The separation by distillation is used to separate liquids from non-volatile solids and to separate liquids having different boiling points. Distillation is a routine activity and can be performed in various devices, all of which are known to the skilled person.

The mixture comprising organic solvent and second solvent from which precipitated polyether polyurethane has been recovered, optionally also contains (dissolved) polyether polyurethane, (dissolved) nylon 6, colorants, additives, degradation products of the organic solvent and/or the second solvent. The mixture comprising organic solvent and third solvent optionally also contains (dissolved) polyether polyurethane, (dissolved) nylon 6, and degradation products of

the organic solvent and/or the third solvent.

In general, the separation of three different liquids by distillation is more complex, requires more energy and requires more equipment than the separation of two different liquids by distillation. The ability to limit the number of different solvents in the process of the invention to two by using the same solvent as second and third solvent therefore is particularly advantageous. In a preferred embodiment of the invention, the second solvent and the third solvent are the same solvent. In a more preferred embodiment, the second solvent and the third solvent are water or an aqueous solution. The use of water as a solvent is advantageous because it is cheap, abundantly available, non-explosive and environmentally benign.

In a preferred embodiment of the invention, the organic solvent is selected from N,N-dimethylformamide (DMF, $(CH_3)_2NC(=O)H$), N,N-dimethylacetamide (DMAc, $CH_3C(=O)N(CH_3)_2$), 1,4-dioxane (dioxane, $C_4H_8O_2$), N-alkyl-2-pyrrolidones such as N-methyl-2-pyrrolidone (NMP, $C_5H_9NO$), tetrahydrofuran (THF; a.k.a. oxolane, $C_4H_8O$). In a more preferred embodiment, the organic solvent is N,N-dimethylacetamide (DMAc, $CH_3C(=O)N(CH_3)_2$).

In another preferred embodiment, the organic solvent is selected from N,N-dimethylformamide (DMF, $(CH_3)_2NC(=O)H$), N,N-dimethylacetamide (DMAc, $CH_3C(=O)N(CH_3)_2$), 1,4-dioxane (dioxane, $C_4H_8O_2$), N-alkyl-2-pyrrolidones such as N-methyl-2-pyrrolidone (NMP, $C_5H_9NO$), tetrahydrofuran (THF; a.k.a. oxolane, $C_4H_8O$) and the second solvent and the third solvent are both water. In a more preferred embodiment of the invention, the organic solvent is N,N-dimethylacetamide (DMAc, $CH_3C(=O)N(CH_3)_2$), and the second solvent and the third solvent are both water. The boiling point of N,N-dimethylacetamide (DMAc) at atmospheric pressure is ca. 165 °C. The boiling point of water at atmospheric pressure is 100 °C.

When only DMAc and water are present in the combined feed to solvent distillation section [δ], the volatility difference between the two is large and separation by distillation has been found to be rather easy. In such situations continuous separation of the combined feed to solvent distillation section [δ] may be realized in a single distillation column, that is operated in a continuous mode. In such a distillation column the water is distilled from the top of the column in a gaseous state, and DMAc is withdrawn from the bottom of the column in a liquid state. When besides DMAc and water also non-volatile compounds (like polyether polyurethane and nylon 6) are present in the combined feed to solvent distillation section [δ], the lay-out of the solvent distillation required for the continuous separation into DMAc, water and non-volatile compounds is more extended. A straightforward lay-out for this type of separation by distillation comprises two distillation columns that are operated in series. The feed (in liquid state) that consists of DMAc, water and the non-volatile compounds is charged to the first distillation column. The water is distilled from the top of the first distillation column in a gaseous state, and a mixture of DMAc and the non-volatile compounds are withdrawn from the bottom of the first distillation column (in a liquid state, or as a slurry). The bottom flow of the first column is charged to the second distillation column. The DMAc is distilled from the top of the second distillation column in a gaseous state, and the non-volatile compounds are withdrawn from the bottom of the second distillation column. In practice a mixture of DMAc and non-volatile compounds may be withdrawn from the bottom of the second distillation column in order to reduce the risk of clogging of the second distillation column. A squeezing (column) unit, that recovers DMAc from the bottom flow of the second distillation column, may be added to enhance the overall recovery of DMAc.

As an alternative for the separation by distillation of two distillation columns that are operated in series, a single distillation column with side withdrawal that is operated in a continuous mode can be chosen. In such a distillation column, the water is distilled from the top of the distillation column in a gaseous state, the DMAc is discharged as a liquid side stream and the non-volatile compounds are withdrawn from the bottom of the second distillation column. Optionally, the bottom flow is charged to a squeezing (column) unit.

As an alternative for charging the feed to the distillation column in liquid state, the charging to the first distillation column can be done in the gaseous state. In such a case the combined feed to solvent distillation section [δ] with the exception of the non-volatile compounds is evaporated in a feed evaporator prior to charging to the first distillation column. The non-volatile compounds are discharged from the feed evaporator. This alternative has the advantage that less fouling occurs in the distillation columns. Optionally, the squeezing column can be combined with the feed evaporator. Generally, DMAc comprising process flows contain certain amounts of acetic acid and dimethylamine, due to, e.g., the degradation of DMAc. This is also the case for the combined feed to solvent distillation section [δ].

The boiling point of pure dimethylamine at atmospheric pressure is ca. 7 °C. Dimethylamine dissolves very well in water.

The boiling point of pure acetic acid at atmospheric pressure is ca. 118 °C.

However, acetic acid and DMAc form a high-boiling azeotrope (with a composition of ca. 21 wt.% acetic acid and ca. 79 wt.% N,N-dimethylacetamide at atmospheric pressure) and the atmospheric boiling point is ca. 171 °C, which is just somewhat higher than the atmospheric boiling point of N,N-dimethylacetamide. A high-boiling azeotrope (or constant boiling point mixture) is a mixture of two liquids that has a boiling point that is higher than the boiling points of the two individual liquids (in pure form).

If the pressure is reduced, the azeotropic point of the acetic acid and N,N-dimethylacetamide mixture shifts to higher concentrations of the low boiling component (which is acetic acid).

At 6.7 kPa a high-boiling azeotrope with a composition of ca. 30 wt.% acetic acid and ca. 70 wt.% N,N-dimethylacetamide is formed with a boiling point of ca. 93 °C.

At 1.3 kPa a high-boiling azeotrope with a composition of ca. 42 wt.% acetic acid and ca. 58 wt.% N,N-dimethylacetamide is formed with a boiling point of ca. 75 °C. Preferably, the degradation products are removed from the solvents that are re-used in the process. This has the advantage that an accumulation of degradation products is prevented. In a preferred embodiment, degradation products of the organic solvent are removed prior to charging the separated organic solvent that is discharged from the solvent distillation section [δ] in step b.10) to the dissolution section [α] in step b.1). However, DMAc and acetic acid cannot be separated by simple distillation due to the formation of the maximum azeotrope.

To the man skilled in the art, several techniques are known to break an azeotrope in distillation.

One group of techniques is based on the addition of another compound which changes the molecular interactions and eliminates the N,N-dimethylacetamide-acetic acid maximum azeotrope. Chlorobenzene, toluene, ethylbenzene and xylene are examples of compounds that break this maximum azeotrope. Addition of one of these compounds to a mixture of N,N-dimethylacetamide and acetic acid results in the formation of a new azeotrope of that compound and acetic acid that can be removed by evaporation. Subsequently, the resulting mixture of that compound and acetic acid is separated.

Another group of techniques, often called pressure-swing distillations, is based on the fact that the maximum azeotrope is pressure dependent. In such systems, two distillation columns are operated at different pressure levels. The feed mixture of N,N-dimethylacetamide and acetic acid is charged to a first distillation column that is operated at a certain pressure. The bottom flow of this first distillation column is charged to the second distillation column. The bottom flow of the second distillation column is charged to the first distillation column. As top products of the distillation columns high purity N,N-dimethylacetamide and acetic acid are obtained. A major advantage of pressure-swing distillations is the absence of foreign compounds.

Finally, there is a group of techniques that converts acetic acid into another component (e.g., salt formation due to addition of caustic) or that selectively adsorbs acetic acid from the mixture of N,N-dimethylacetamide and acetic acid.

When besides N,N-dimethylacetamide (DMAc), water and non-volatile compounds (like polyether polyurethane and polyamide 6), also acetic acid and dimethylamine are present in the combined feed to solvent distillation section [δ], the lay-out of the solvent distillation required for the continuous separation into DMAc, water, non-volatile compounds, acetic acid and dimethylamine is even more extended.

The optimal lay-out is very much dependent on both the composition and the volume flow of the combined feed to solvent distillation section [δ]. The lay-out may contain a combination of various separation solutions that were described before.

In case the fraction of acetic acid in the combined feed to solvent distillation section [δ] is rather low, the solvent distillation section [δ] may comprise two distillation columns that are operated in series, followed by a neutralizer-evaporation step. The feed that consists of DMAc, water, non-volatile compounds, acetic acid and dimethylamine is charged to the first distillation column. The water and dimethylamine are distilled from the top of the first distillation column in a gaseous state, and a mixture of DMAc, acetic acid and the non-volatile compounds are withdrawn from the bottom of the first distillation column (in a liquid state, or as a slurry). The water vapors from the top of the first distillation column are condensed. The resulting liquid phase contains water and dissolved dimethylamine. In a dimethylamine stripping unit the dimethylamine can be removed from this liquid phase, whereby water is obtained that is (almost) free of dimethylamine. The bottom flow of the first column is charged to the second distillation column. The DMAc is distilled from the top of the second distillation column in a gaseous state, and a mixture of DMAc, acetic acid and the non-volatile compounds are withdrawn from the bottom of the second distillation column. The bottom flow of the second column is charged to the neutralizer-evaporation unit. To the neutralizer-evaporation unit a base, e.g., aqueous NaOH, is charged to neutralize the acetic acid. Due to evaporation, gaseous DMAc is discharged from the neutralizer-evaporation unit. If present also water is evaporated. A mixture of neutralized acetic acid (acetic acid containing salt) and non-volatile compounds is discharged from the bottom of the neutralizer-evaporation unit. In practice a mixture of DMAc, neutralized acetic acid (acetic acid containing salt) and non-volatile compounds may be withdrawn from the bottom of the neutralizer-evaporation unit in order to reduce risk of clogging of the neutralizer-evaporation unit.

The DMAc that is recovered by distillation is discharged from solvent distillation section [δ] and charged to dissolution section [α].

Preferably, water that is recovered in solvent distillation section [δ], optionally after removal of dimethylamine, is re-used in the process of the invention. Preferably, water is used as second solvent and/or as third solvent.

<u>The charging step c.1.)</u>

In step c.1) of the invention, the resulting nylon 6 rich stream is charged to the depolymerization section [C] wherein the nylon 6 content of the nylon 6 rich stream is at least 85 %, preferably at least 90 % by weight on dry-weight

basis. This has the advantage that the nylon 6 rich stream that is charged to the depolymerization section [C] is less contaminated with foreign materials, which improves the yield and the purity of the ε-caprolactam that is produced in the plant of the invention configured to carry out the process of the invention. Other advantages are that a nylon 6 rich stream will require less depolymerization agent, will produce less waste and will require less energy in the depolymerization section [C]. And finally, a nylon 6 rich stream will cause less operational problems, like fouling and clogging of equipment. The depolymerization section [C] comprises one or more depolymerization reactors that are operated in series and/or in parallel.

Optionally, the nylon 6 rich stream is mechanically compressed into a smaller volume prior to being charged to the depolymerization section [C]. This has the advantage that less volume is needed for intermediate storage and transport and can also facilitate dosing to the depolymerization section [C].

Optionally, the nylon 6 rich stream is compressed into particles with an increased density prior to being charged to the depolymerization section [C]. This can be achieved, e.g., by mechanical compaction or by extrusion of melted material through a die followed by cooling and cutting it to size. Compression of the nylon 6 rich stream into particles has the advantage of an increased bulk density, which reduces the costs of intermediate storage and transportation. Apart from density increase, pelletization also offers other benefits, such as a homogeneous shape and structure that is advantageous for (automated) feeding into the depolymerization section [C].

Optionally, the nylon 6 rich stream is charged to a smelter (e.g., an extruder). In the smelter the nylon 6 rich stream is melted. Preferably, the resulting polymer melt is filtered. This has the advantage that solid impurities are removed. The melted and optionally filtered polymer melt is cooled and then fed to a pelletizer to obtain pellets. These pellets are charged to the depolymerization section [C].

The dimensions and shape of the pellets (also often called granules) can be chosen within wide limits. In general, pellets are cylindrical in shape (originating from thin strands that are chopped into pieces). Other shapes like (non-perfect) spheres, however, are also possible. The dimensions of the pellets can be chosen within wide limits. Pellets can have a diameter that ranges from 1 to 10 mm, preferably from 2 to 5 mm, more preferably from 3 to 5 mm. In a preferred embodiment, pellets have a length that ranges from 1 to 50 mm, preferably from 2 to 25 mm and more preferably from 5 to 15 mm.

Optionally, the nylon 6 rich stream that is discharged from separation section [B] is dried prior to being charged to depolymerization section [C]. This has the advantage that less or no solvent is introduced in the depolymerization section [C]. Solvent introduced in the depolymerization section [C] can negatively influence the depolymerization process (e.g., lead to reduced depolymerization reaction rates, higher consumption of catalyst, higher energy consumption, and the vapor stream comprising ε-caprolactam and water that is obtained in the depolymerization section [C] can contain more impurities).

The nylon 6 rich stream is preferably fed to the depolymerization reactor(s) as a solid phase or as a melt. Preferably, the nylon 6 rich stream is charged as a melt. Feeding as a melt may be achieved by using an extruder, gear pump, or other means known by the skilled person.

The feeding of the nylon 6 rich stream to the depolymerization reactor(s) may be realized by continuous or by intermittent dosing of the nylon 6 rich stream.

<u>The depolymerization step c.2)</u>

In the depolymerization section [C], the nylon 6 rich stream is depolymerized to form ε-caprolactam. The formed ε-caprolactam is discharged from the depolymerization section [C] as an ε-caprolactam comprising stream.

The depolymerization of the nylon 6 rich stream is achieved by increasing the temperature of the nylon 6 rich stream to a temperature of at least 180 °C, but not higher than 400 °C in the depolymerization section [C]. The preferred temperature range for the depolymerization reaction is from 200 °C to 350 °C, more preferably from 220 °C to 340 °C, and most preferably from 240 °C to 325 °C. Experiments have shown that in these temperature ranges the depolymerization is particularly effective and less side reactions of polyamide 6 and ε-caprolactam and reactions of impurities occur.

Generally, the rate of ε-caprolactam formation increases at elevated temperatures. Temperatures lower than 400 °C are preferred since at temperatures above 400 °C side reactions of nylon 6 and reactions of impurities occur more frequently which will result in formation of a more diverse set of impurities. Part of these impurities will end-up in the ε-caprolactam comprising product stream that is discharged from the depolymerization reactor section [C]. In a preferred embodiment of the invention, the depolymerization of the nylon 6 rich stream is conducted at temperatures ranging of from 220 °C to 340 °C or 240 °C to 325 °C. This temperature range allows production of particularly pure ε-caprolactam.

The pressure in the depolymerization section [C] can vary and may range from 1 kPa to 100 MPa, preferably from 10 kPa to 5 MPa, more preferably from 25 kPa to 2 MPa, most preferably from 50 kPa to 1 MPa. This pressure range allows production of particularly pure ε-caprolactam.

The depolymerization of the nylon 6 rich stream can be achieved in the presence or in the absence of a solvent.

Preferably, the depolymerization of the nylon 6 rich stream is achieved in the presence of water as solvent. In this case, the water is preferably in the form of steam, in particular superheated steam.

Preferably, depolymerization will be complete in 0.1 hour to 24 hours, more preferably in 0.5 hour to 6 hours.

Feeding the water as steam to the depolymerization reactor allows, optionally without further heating, to obtain a vapor stream comprising ε-caprolactam and water. The weight to weight ratio of ε-caprolactam to water in this vapor stream can be adjusted by modifying the amount of steam that is fed to the nylon 6 rich stream in the depolymerization section [C]. In a preferred embodiment, the depolymerization in step c.2) is performed in the presence of water, whereby the ε-caprolactam comprising stream is a vapor stream comprising ε-caprolactam and water in a weight to weight ratio of from 1:1 to 1:50, preferably from 1:2 to 1:15, more preferably from 1:2 to 1:10 and most preferably from 1:3 to 1:8. Within these ranges, a particularly economically efficient process can be conducted.

Preferably, the ε-caprolactam in the vapor stream comprising ε-caprolactam and water has a partial pressure of 0.1 kPa to 1 MPa, more preferably of 0.3 kPa to 0.5 MPa, and most preferably of 1 kPa to 0.1 MPa.

During the depolymerization reaction, decomposition products may be formed including oligomers of ε-caprolactam. In addition, the feed stream of the material derived from nylon 6 comprising multi-component material may also contain other components, i.e., impurities such as non-nylon 6 compounds and residues of solvents(s) applied in the pre-treatment that remain stable, react or decompose under the depolymerization conditions. Thus, in case water is used as solvent then the vapor stream which is removed from the depolymerization section [C] does not only comprise water and ε-caprolactam, but also impurities.

Preferably, superheated steam with a temperature between 100 °C and 600 °C is charged to the depolymerization reactor(s). Preferably, the superheated steam that is charged to the depolymerization reactor(s) has a temperature of at least the melting temperature of nylon 6. Preferably, the energy content of the superheated steam that is charged to the depolymerization reactor(s) is sufficiently high so that no other heat input is needed for performing the depolymerization reaction and evaporating the formed ε-caprolactam. In another preferred embodiment, the depolymerization section [C] is charged with superheated steam having a temperature ranging of from 220 °C to 575 °C. In an even more preferred embodiment, the depolymerization section [C] is charged with super-heated steam having a temperature ranging of from 275 °C to 500 °C. In another preferred embodiment, a part of the heat input that is needed for performing the depolymerization reaction and evaporating the formed ε-caprolactam is introduced via the walls of the depolymerization reactor(s).

Generally, the mass of the vapor stream, which is removed from the depolymerization section [C], is less than the mass of the total feed to the depolymerization section. The total feed to the depolymerization section [C] comprises nylon 6 rich stream and optionally solvent, catalyst, additional agents and/or depolymerizing agents. Thus, without any additional measures, there will be an accumulation of material (often called ’residual material’) in the depolymerization section [C]. Preferably, another stream is discharged from the depolymerization section [C]. This has the advantage that the accumulation of material in the depolymerization section [C] is reduced or avoided. The additional stream can comprise impurities present in the nylon 6 rich stream, non-depolymerized nylon 6, non-evaporated ε-caprolactam, catalyst(s) and compounds that were formed under the depolymerization conditions, like mono-, di- and/or triammonium phosphate, in case phosphoric acid is used as depolymerization catalyst. In a preferred embodiment, a stream comprising mono-, di- and/or triammonium phosphate is discharged from the depolymerization section [C]. Even more preferably, this stream, which is intermittently or continuously discharged from the depolymerization section [C], comprises mono-, di- and/or triammonium phosphate in a weight fraction of 0.01 to 50 % by weight, preferably from 0.1 to 25 % by weight, more preferably from 0.5 to 10 % by weight, most preferably from 0.5 to 5 % by weight.

The depolymerization of the nylon 6 rich stream in the presence of steam can be performed with the presence of additional depolymerizing agents, such as ammonia. The concentration of ammonia in the depolymerization section [C] can vary. Thus, in case ammonia is present in the depolymerization section [C], then the vapor stream which is removed from the depolymerization section [C] does not only comprise ε-caprolactam and impurities, but may also comprise ammonia.

Most preferably, the depolymerization is carried out in the presence of a catalyst. Preferably, the used catalyst is a (Lewis or Brønsted) acid or base. The acid catalyst can in particular be selected from the group consisting of orthophosphoric acid, p-toluenesulfonic acid, boric acid, sulfuric acid, organic acid, organic sulfonic acid including xylene sulfonic acid, 4-sulfo isophthalic acid and other sulfonated aromatic hydrocarbons, solid acids, salts of the aforementioned acids, $Al_2O_3$ and $SiO_2$, and combinations thereof. The base catalyst can, e.g., be selected from the group consisting of alkali hydroxide, alkali salt, alkaline earth hydroxide and alkaline earth salts, organic bases and solid bases, and combinations thereof. Preferably, orthophosphoric acid, boric acid, organic acid, alkali hydroxides and alkali salts are used as catalysts. More preferably, orthophosphoric acid, sodium phosphate, potassium phosphate, sodium hydroxide, potassium hydroxide, sodium carbonate, potassium carbonate, sodium hydrogen carbonate and potassium hydrogen carbonate are used as catalysts. Still more preferably, orthophosphoric acid, p-toluenesulfonic acid, boric acid and sodium hydroxide are used as catalysts. In one particularly preferred embodiment, orthophosphoric acid is used as catalyst for the depolymerization, in another, p-toluenesulfonic acid is used.

In another preferred embodiment, however, no catalyst is used for the depolymerization of the nylon 6 rich stream. This has the advantage of lower costs (both for the catalyst and the disposal of catalyst waste). However, higher temperatures (and pressures) are usually required compared to depolymerizations of the nylon 6 rich stream that are carried out in the presence of a catalyst.

The advantage of using a catalyst (and especially of orthophosphoric acid) is that the depolymerization reaction already starts at lower temperatures and can be performed under atmospheric conditions. A suitable concentration of catalyst used for the depolymerization of nylon 6 to ε-caprolactam is known to the skilled person and can easily be determined by routine experimentation. If the concentration of the used catalyst is too low, the reaction rate is slow. On the contrary, if the concentration of the used catalyst is too high, the reaction is fast, but also side reaction(s) increase. Moreover, the catalyst costs are increased, which is economically disadvantageous. Preferably, the catalyst content is from 0.01 to 100 % by weight relative to the nylon 6 contained in the depolymerization reactor. Even more preferably, the catalyst content is from 0.1 to 50 % by weight. The optimum catalyst concentration depends on the type of catalyst that is applied for the depolymerization of nylon 6. For the catalyst orthophosphoric acid, the preferred content is from 0.1 to 25 % and more preferred from 1 to 20 % by weight. The preferred content for the catalyst p-toluenesulfonic acid is from 10 to 35 % by weight and the more preferred content from 15 to 30 % by weight.

The depolymerization of nylon 6 can be performed in a batch mode, in a semi-continuous mode or in a continuous mode, all of which are known to the skilled person. The terms "batch mode", "semi-continuous mode" and "continuous mode", as used herein, refer to the mode in which the nylon 6-containing feedstock, i.e., the nylon 6 rich stream, and optionally catalyst are charged to the depolymerization reactor and to the mode in which the residual material is discharged from the depolymerization reactor.

In a preferred embodiment, the nylon 6 depolymerization is performed in the batch mode. In the batch mode, the feedstock, i.e., the material derived from nylon 6 comprising multi-component material, and optionally catalyst are initially charged to the depolymerization reactor. Subsequently, superheated steam is charged to the depolymerization reactor and ε-caprolactam is discharged from the depolymerization reactor as vapor stream comprising ε-caprolactam and water. Next, charging of the superheated steam to the depolymerization reactor is interrupted. After optionally removing residual material from the depolymerization reactor, a new cycle is started by charging feedstock (and optionally catalyst) to the depolymerization reactor. In a preferred embodiment, residual material is not removed in between every cycle.

In a particular advantageous embodiment, the nylon 6 depolymerization is performed in the continuous mode. In the continuous mode, the nylon 6-containing feedstock (and optionally catalyst) is continuously charged to the depolymerization reactor. At the same time, superheated steam is continuously charged to the depolymerization reactor and ε-caprolactam is continuously discharged from the depolymerization reactor as vapor stream comprising ε-caprolactam and water. Optionally, the catalyst is continuously or intermittently charged to the depolymerization reactor. In addition, residual material is continuously discharged from the depolymerization reactor. Preferably, the nylon 6 rich stream is charged as a melt. Preferably, the catalyst is charged as a melt, a slurry or a solution.

In another preferred embodiment, the nylon 6 depolymerization is performed in the semi-continuous mode. In the semi-continuous mode, nylon 6-containing feedstock (and optionally catalyst) is intermittently charged to the depolymerization reactor, while superheated steam is continuously charged to the depolymerization reactor and ε-caprolactam is continuously discharged from the depolymerization reactor as a vapor stream comprising ε-caprolactam and water. Residual material is intermittently discharged from the depolymerization reactor in the semi-continuous mode of nylon 6 depolymerization.

The recovery step c.3)

In the recovery section [D], ε-caprolactam is recovered from the ε-caprolactam comprising stream that is discharged from the depolymerization section [C]. This stream comprises ε-caprolactam and impurities. Preferably, this recovery is performed by a (partial) condensation of the ε-caprolactam comprising stream.

Preferably, in case no solvent is charged to the depolymerization section [C], the ε-caprolactam that is obtained by condensation is dissolved in water, whereby an ε-caprolactam-rich phase is obtained. This ε-caprolactam-rich phase also comprises impurities.

Preferably, in case water is charged to the depolymerization section [C] as solvent, the ε-caprolactam comprising stream that is discharged from the depolymerization section [C] comprises ε-caprolactam, water and impurities. Water can be charged as a liquid or in the form of steam. Preferably, water is charged in the form of steam. The ε-caprolactam can be separated from the ε-caprolactam comprising stream that is discharged from the depolymerization section [C] by sending this vapor or gaseous stream from the depolymerization reactor, preferably overhead, to a (preferably partial) condenser to obtain a condensate containing ε-caprolactam. Preferably, the ε-caprolactam is separated from the remaining components of the vapor stream by sending the product stream from the depolymerization reactor, preferably overhead, to a distillation column from which a water-rich phase is obtained as top product and an ε-caprolactam-rich

phase as bottom product.

The ε-caprolactam recovered in the recovery section [D] is crude since it contains impurities such as nylon 6 decomposition products or other impurities stemming from (decomposition products of) non-nylon 6 components of the material derived from the nylon 6 rich stream nylon 6 rich stream that is charged to the depolymerization section [C]. The crude ε-caprolactam that is recovered in step c.3) comprises water and ε-caprolactam, preferably it is an aqueous solution comprising ε-caprolactam. Thus, the crude ε-caprolactam recovered in the recovery section [D] requires additional purification to yield high purity ε-caprolactam. "Crude" as used herein can therefore be defined as being less pure, i.e., containing more impurities, than the purified ε-caprolactam obtained as the product of the process of the invention.

Preferably, the crude ε-caprolactam comprises ε-caprolactam in the range of from 6 to 95 % by weight, more preferably from 20 to 90 % by weight, and most preferably from 35 to 80 % by weight. The remainder is mainly water.

The purifying step c.4)

In step c.4), the crude ε-caprolactam which is obtained in the recovery section [D] is purified in the purification section [E] to yield high purity ε-caprolactam.

Optionally, the crude ε-caprolactam is filtered before being charged to the purification section [E]. The filtration ensures the removal of undissolved impurities which could otherwise hinder the further purification process.

Optionally, oil is separated from the crude ε-caprolactam before being charged to the purification section [D]. The oil separation ensures the removal of impurities which could otherwise hinder the further purification process.

Preferably, purified ε-caprolactam is obtained by purifying the crude ε-caprolactam obtained in the recovery section [D] in the purification section [E] via crystallization of ε-caprolactam from a solution comprising ε-caprolactam and impurities at a temperature of 10 °C to 95 °C, more preferably at a temperature of 20 °C to 85 °C.

The process of the invention is performed in a plant, wherein the plant comprises purification section [E], wherein the purification comprises the step of
c.4)(iv) obtaining purified ε-caprolactam by crystallization of ε-caprolactam from a solution comprising ε-caprolactam and impurities at a temperature of 10 °C to 95 °C.

Preferably, the process of the invention is performed in a plant, wherein the plant comprises purification section [E], wherein the purification further comprises the step of

> c.4)(i) extracting the crude ε-caprolactam with an organic solvent, whereby an aqueous phase and an organic phase are obtained, and wherein the organic phase comprises the organic solvent, ε-caprolactam and impurities;
> c.4)(ii) optionally, switching the solvent by replacing the organic solvent at least partially with water, whereby an aqueous phase comprising water, ε-caprolactam and impurities with lower- or higher-boiling points than ε-caprolactam is obtained and wherein the solvent switch step (ii) is selected from a process based on back-extraction with water, and a process based on solvent swap distillation, in which the organic solvent is distilled off and water is charged; and
> c.4)(iii) obtaining purified ε-caprolactam by distillative removal of impurities with lower- or higher-boiling points than ε-caprolactam.

High purity ε-caprolactam is obtained from the crude ε-caprolactam by first extracting in step c.4)(i) the crude ε-caprolactam with an organic solvent, whereby an aqueous phase and an organic phase comprising the organic solvent, ε-caprolactam and impurities are obtained. The organic solvent with which the crude ε-caprolactam is extracted is preferably an aromatic hydrocarbon, a halogenated hydrocarbon and/or a $C_4$-$C_{10}$ aliphatic or $C_4$-$C_{10}$ cycloaliphatic alcohol. Optionally, the organic solvent with which the crude ε-caprolactam is extracted is preferably a mixed extractant, i.e., it can consist of one or more organic solvents and optionally further diluents. Preferably, the one or more organic solvents is independently selected from an aromatic hydrocarbon, a halogenated hydrocarbon and/or a $C_4$-$C_{10}$ aliphatic or cycloaliphatic alcohol, and a $C_5$-$C_8$ alkane or $C_5$-$C_8$ cycloalkane. Particularly good purification results are achieved if the organic solvent for extraction of the crude ε-caprolactam is selected from the group consisting of cyclohexane, benzene, toluene, methylene chloride, chloroform, trichloroethane, 4-methyl-2-pentanol (a.k.a. MIBC, methyl isobutyl carbinol), 1-octanol, 2-ethylhexanol and mixtures thereof. More preferably, the organic solvent for extraction of the crude ε-caprolactam is selected from the group consisting of benzene, toluene, cyclohexane, alcohols, and mixtures thereof. Still more preferably, the organic solvent for extraction of the crude ε-caprolactam is selected from the group consisting of toluene, cyclohexane, 1-octanol, 2-ethylhexanol and mixtures thereof. Preferably, the weight ratio of organic solvent to ε-caprolactam is from 0.01:1 to 50:1, preferably from 0.05:1 to 20:1, more preferably from 0.1:1 to 10:1, and most preferably from 0.1:1 to 5:1.

In another embodiment, the extraction with organic solvent in step c.4)(i) is carried out in a counter-current operated extraction column whereby the crude ε-caprolactam to be purified is introduced at the top and the organic solvent at the bottom of the column. The extraction results in an aqueous phase and an organic phase comprising the

organic solvent, ε-caprolactam and impurities.

Optionally, the organic phase comprising the organic solvent, ε-caprolactam and impurities is washed with water or with an aqueous alkaline solution before entering step c.4)(ii). If washing is performed with an aqueous alkaline solution, the alkaline solution is preferably an aqueous solution comprising an alkali metal hydroxide and/or alkali metal carbonate, preferably sodium hydroxide or potassium hydroxide. Said alkali metal hydroxide solution preferably comprises 0.5 wt.% to 2.0 wt.% of sodium hydroxide or potassium hydroxide.

The skilled person can determine by routine experimentation the amount of water or aqueous alkaline solution necessary for efficient washing of the organic phase comprising the organic solvent, ε-caprolactam and impurities. Preferably, this amount is between 0.1 vol.% and 5 vol.% relative to the amount of organic solvent in the to-be-washed organic phase.

In another preferred embodiment, the washing of the organic phase comprising the organic solvent, ε-caprolactam and impurities with water or aqueous alkaline solution is carried out in a counter-current operated washing column whereby the organic phase comprising the organic solvent, ε-caprolactam and impurities is introduced at the bottom and the water or aqueous alkaline solution at the top of the column. The washing results in a washed organic phase comprising the organic solvent, ε-caprolactam and impurities and a residue-comprising phase. The residue-comprising phase then contains water, impurities and ε-caprolactam.

In a preferred embodiment, the organic phase comprising the organic solvent, ε-caprolactam and impurities that is optionally washed is subsequently extracted with water, whereby an ε caprolactam-water phase is obtained. Preferably, this ε-caprolactam-water phase is then stripped and/or distilled to remove residual solvent. Although the amount of the water used for the recovery of ε-caprolactam can vary, the amount of the water used is 0.5 to 20 times, preferably 0.75 to 10 times, and more preferably 1 to 5 times by weight based on the recovered ε-caprolactam.

In another preferred embodiment, the extraction with water is carried out in a counter-current operated extraction column, the ε-caprolactam containing phase to be purified being introduced at the bottom and the water at the top of the column. The extraction results in an ε-caprolactam-water phase and in a solvent phase that comprises impurities. Usually the solvent phase that comprises impurities is, optionally after purification, preferably by distillation, re-used.

The ε-caprolactam-water phase, that was optionally stripped and/or distilled to remove residual solvent, is concentrated by evaporation of water so that a concentrated aqueous ε-caprolactam phase is obtained. The ε-caprolactam content of this concentrated aqueous ε caprolactam phase is usually between 60 wt.% and 99.9 wt.% relative to the entire phase.

In another preferred embodiment, organic solvent is evaporated from the organic phase comprising organic solvent, ε-caprolactam and impurities that is optionally washed instead of being extracted with water. Any suitable evaporation vessel may be used, for example a column. Preferably the evaporation is performed in the presence of water. More preferably, the evaporation is performed as an azeotropic distillation in which case the organic solvent is evaporated as an azeotropic mixture. The evaporation results in ε-caprolactam product. Preferably, the ε-caprolactam product is an aqueous ε-caprolactam phase. The ε-caprolactam content of this aqueous ε-caprolactam phase is usually between 40 wt.% and 99.9 wt.% relative to the entire phase.

In another preferred embodiment, prior to the distillative removal in step c.4)(iii) of the process of the invention, an oxidant, e.g., potassium permanganate, sodium permanganate, and/or hydrogen peroxide, is added to the ε-caprolactam-water phase. Most preferably, potassium permanganate is used as oxidant.

Preferably, the oxidant is added as solid, as a slurry or in the form of an aqueous solution to the ε-caprolactam-water phase, so that a dilute aqueous solution is obtained during purification by oxidation. The skilled person can determine by routine experimentation the amount of oxidant necessary for efficient oxidation of the ε-caprolactam-water phase. The exact amount of oxidant is, amongst others, very much dependent on the composition of the polyamide 6 rich streams that are charged to the depolymerization section in this process of the invention. Preferably, the amount of oxidant is between 0.01 and 5 % by weight relative to the amount of ε-caprolactam dissolved in the to-be-oxidized aqueous phase.

The temperature used for oxidation of the aqueous solution in the process of the invention can vary. Preferably, oxidation of this aqueous solution prior to the distillative removal in step c.4)(iii) with an oxidant is performed at a temperature ranging from 20 °C to 85 °C, more preferably ranging from 30 °C to 80 °C, wherein the oxidant is selected from the group consisting of potassium permanganate, sodium permanganate and hydrogen peroxide and combinations thereof, in particular potassium permanganate.

The length of time used for oxidation with an oxidant can vary. Preferably, in the process of the invention, prior to the distillative removal in step c.4)(iii), oxidation of the ε-caprolactam-water phase with an oxidant is performed for 1 minute to 24 hours, more preferably for 2 minutes to 6 hours, and most preferably for 5 minutes to 2 hours.

The concentration of ε-caprolactam in the ε-caprolactam-water phase used for oxidation with an oxidant can vary. Preferably, the aqueous solution used for oxidation comprises a weight to weight ratio of ε-caprolactam to water from 5 : 1 to 1 : 5, more preferably from 3 : 1 to 1 : 3 and, most preferably from 2 : 1 to 1 : 2. Optionally, prior to the addition of the oxidant to the aqueous phase, the weight to weight ratio of ε-caprolactam to water is adapted. Preferably,

the weight to weight ratio of ε-caprolactam to water is adapted by either addition of water or by removal of water.

In case potassium permanganate and/or sodium permanganate is used as oxidant, solid manganese(IV) oxide ($MnO_2$) particles are formed as reaction product. The skilled person can determine by routine experimentation the optimal solid-liquid filtration procedure for efficient removal of solid manganese(IV) oxide particles from the aqueous phase after oxidation. The usage of filter aids, like activated carbon or kieselguhr particles, to improve the filtration procedure are in this regard common practice.

The aqueous ε-caprolactam phase is optionally hydrogenated and if the aqueous ε-caprolactam phase is hydrogenated, then preferably in the presence of a hydrogenation catalyst known per se. The hydrogenation can be carried out as for example described in EP635487.

In a preferred embodiment of the invention, prior to the crystallization in step c.4)(iv), the aqueous solution comprising water, ε-caprolactam and impurities is hydrogenated in the presence of a hydrogenation catalyst. The hydrogenation catalysts may be any known heterogeneous hydrogenation catalyst. Examples of such catalysts are ruthenium on aluminum oxide, rhodium on aluminum oxide, platinum on carbon, palladium on carbon, Raney nickel, nickel on silica and nickel on aluminum oxide. Preferably, use is made of nickel-containing catalysts. Suitable nickel catalysts as a rule have a nickel content between 5 and 80 wt.%, relative to the metal and the support. Besides nickel the catalyst may contain some activators such as Zr, Mn, Cu or Cr. The activator content is generally between 1 and 20 wt.%. If palladium-containing heterogeneous catalysts are used, the palladium content will generally be between 0.01 and 10 wt.%.

The heterogeneous catalyst can be contacted with the hydrogen-containing reaction mixture in various ways. Hydrogenation may for instance take place in a stirred tank reactor in which the catalyst particles are suspended in the mixture to be purified (slurry phase process). In another embodiment, the hydrogenation is effected in a fixed-bed reactor with the catalyst being fixed in the reactor.

The hydrogenation can be performed in a three-phase system (gas, liquid, solid) that comprises of an aqueous ε-caprolactam mixture, gaseous hydrogen and heterogeneous hydrogenation catalyst. Alternatively, the hydrogenation can be performed in a two-phase system (liquid, solid) that comprises of an aqueous ε-caprolactam mixture, that is completely or partially saturated with hydrogen, and heterogeneous hydrogenation catalyst. Dissolution of the hydrogen in the water-ε-caprolactam mixture to obtain a mixture that is completely or partially saturated with hydrogen can be effected by any process that is known to one skilled in the art.

The hydrogenation temperature is generally between 20 °C and 160°C. The hydrogenation pressure is generally between 0.1 and 15 MPa.

Hydrogenation of water-ε-caprolactam mixtures are carried out to hydrogenate the unsaturated compounds present in the impure ε-caprolactam. The presence of these unsaturated compounds are disadvantageous because they can impair the physical-mechanical properties of the nylon 6 made by polymerizing ε-caprolactam. The saturated compounds formed by hydrogenation do not adversely influence these physical-mechanical properties of the nylon 6 and moreover these compounds are more easily removed in for example a distillation step and/or a crystallization step following the hydrogenation step.

Preferably, water is evaporated from the, optionally hydrogenated, aqueous ε-caprolactam phase. Following the hydrogenation and/or evaporation of water, the aqueous ε-caprolactam phase is distilled to recover high purity ε-caprolactam and a distillation residue.

In step c.4)(iii) of the process of the invention, the optionally washed organic phase comprising the organic solvent, ε-caprolactam and impurities is subsequently distilled. Preferably, distillation of the optionally washed organic phase comprising the organic solvent, ε-caprolactam and impurities is carried out at a reduced pressure. In one embodiment, the distillation is effected at a pressure of less than 350 kPa, preferably less than 50 kPa, more preferably less than 20 kPa and most preferably less than 10 kPa. Preferably, the distillation temperature at the bottom of the distillation column is between 100 °C and 200 °C and more preferably between 110 °C and 180 °C. The distillation includes the separation of low-boiling organic impurities (having a lower boiling point than ε-caprolactam) and/or separating high-boiling organic impurities (having a higher boiling point than ε-caprolactam) from ε-caprolactam.

In a preferred embodiment, prior to the distillative removal in step c.4)(iii), alkali metal hydroxide, preferably NaOH, is added to the ε-caprolactam comprising phase. Preferably, the amount of NaOH that is added ranges from 0.5 to 100 mmol per kg ε-caprolactam, and more preferably from 2 to 80 mmol per kg ε-caprolactam. Experiments have shown that the addition of an alkali metal hydroxide, in particular NaOH, allows for a particular effective distillative removal of impurities with lower and higher boiling points than ε-caprolactam.

In another preferred embodiment, crude ε-caprolactam obtained from a Beckmann rearrangement of cyclohexanone oxime is also purified in the purification section [E] apart from the crude ε-caprolactam that is recovered in the recovery section. This has the advantage that by introducing recycled ε-caprolactam according to the present invention, the carbon footprint of a plant producing ε-caprolactam *de novo* from a Beckmann rearrangement of cyclohexanone oxime can be reduced.

In step c.4)(iv) of the process of the invention, the solution comprising ε-caprolactam and impurities is crystallized

to obtain purified ε-caprolactam at a temperature of 10 °C to 95 °C.

Preferably, the solution comprising ε-caprolactam and impurities that is crystallized in step c.4)(iv) is the crude ε-caprolactam obtained in the recovery section [D].

More preferably, the solution comprising ε-caprolactam and impurities from which purified ε-caprolactam is obtained by crystallization in step c.4)(iv) is the organic phase comprising the organic solvent, ε-caprolactam and impurities, that is optionally washed with water or with an aqueous alkaline solution, that is obtained by extraction in step c.4d)(i).

Even more preferably, the solution comprising ε-caprolactam and impurities from which purified ε-caprolactam is obtained by crystallization in step c.4)(iv) is the aqueous phase comprising water, ε-caprolactam and impurities with lower- or higher-boiling points than ε-caprolactam that is obtained by solvent switching in step c.4d)(ii).

Most preferably, the solution comprising ε-caprolactam and impurities from which purified ε-caprolactam is obtained by crystallization in step c.4)(iv) is the phase comprising ε-caprolactam and impurities that is obtained by distillation under vacuum conditions in step c.4d)(iii).

Preferably, the ε-caprolactam crystallization process in step d(iv) comprises the following steps:

1. a solution comprising ε-caprolactam and impurities is fed into a crystallizer;
2. in the crystallizer conditions are set such that ε-caprolactam crystals and a mother liquid are formed;
3. the ε-caprolactam crystals and the mother liquid are separated;
4. the mother liquid is recycled.

Crystallization can be applied for the production of ε-caprolactam. It is mainly used for its purification potential and/or product recovery to increase yield. All crystallization processes are based on the formation of a solid crystalline phase from a liquid. In a preferred embodiment, the crystallization in step d)(iv) is performed by either solution crystallization or melt crystallization.

The term solution crystallization is used for crystallization of a compound from a solution that comprises that (impure) compound and to which an auxiliary solvent is added. The auxiliary solvent is water or a non-aqueous solvent. In case the auxiliary solvent is water then the amount of water in the solution can be chosen within wide ranges, preferably the amount of water ranges from 0.5 to 25 wt.%, more preferably from 1 to 9 wt.%. The crystallization temperature can be chosen within wide ranges, preferably, the crystallization temperature ranges from 10 °C to 95 °C, more preferably from 20 °C to 85 °C, even more preferably, the crystallization temperature ranges from 20 °C to 70 °C, most preferably from 30 °C to 65 °C. The crystallized purified ε-caprolactam is recovered from the slurry at a slurry concentration of preferably ranging from 5 to 75 wt.%., more preferably ranging from 10 to 70 wt.%, most preferably ranging from 15 to 50 wt.%. In case the auxiliary solvent is a non-aqueous solvent then the amount of non-aqueous solvent in the solution can be chosen within wide ranges, preferably the amount of non-aqueous solvent ranges from 5 to 95 wt.%, more preferably from 10 to 90 wt.%, most preferably from 30 to 70 wt.%. The crystallization temperature can be chosen within wide ranges, preferably, the crystallization temperature ranges from 10 °C to 95 °C, more preferably from 20 °C to 85 °C, even more preferably, the crystallization temperature ranges from 20 °C to 70 °C, most preferably from 30 °C to 65 °C. The crystallized purified ε-caprolactam is recovered from the slurry at a slurry concentration of preferably from 5 to 75 wt.%, more preferably from 10 to 70 wt.%, most preferably from 15 to 50 wt.%. Examples of non-aqueous solvents include alkanes (like n-hexane, n-heptane, iso-octane, cyclohexane), alcohols (like methanol, ethanol, n-propanol, n-butanol), aromatic hydrocarbons (like benzene, toluene, o-xylene, m-xylene, p-xylene), ammonia, chlorinated hydrocarbons (like tetrachloromethane, chloroform or ethyl chloride), ketones (like acetone or methyl ethyl ketone) and esters (like ethyl acetate), and mixtures of these solvents. Among them, cyclohexane is preferable.

Solution crystallization is usually performed under atmospheric pressure, but may be performed under reduced pressure or under pressurized conditions. In case of solution crystallization the product is crystallized by evaporative crystallization, whereby the solvent is evaporated, or by cooling crystallization, whereby the cooling is obtained by direct cooling, indirect cooling or vacuum cooling, or by a combination of these methods. After the crystallization step, the formed crystals and the mother liquor are separated by e.g., sedimentation, filtration and/or centrifugation. Optionally, the resulting crystals are washed with e.g., a solvent or solvent mixture with a low impurity content. Optionally, the crystallization - separation sequence is repeated several times. The product is obtained as crystals.

The narrow definition of the term melt crystallization is crystallization of a compound from a solution that comprises that (impure) compound without using an auxiliary solvent. In a wider definition of the term melt crystallization is also applied for crystallization from a solution containing low solvent concentrations. Preferably, the solvent concentration in the solution is less than 25 wt %, more preferably less than 10 wt. %, most preferably less than 5 wt. %. Here, we will use the wider definition of melt crystallization, unless explicitly mentioned otherwise. The compound crystals obtained by melt crystallization are separated from the mother liquor and are optionally washed with the melt of pure compound material. The purification efficiency can be further improved by e.g., sweating a.k.a. partial melting, i.e., mildly heating up the crystal layer to nearly its melting temperature causing draining off of entrapped and adherent impure mother liquor. Optionally, the crystallization - separation sequence is repeated several times. Finally, the optionally washed

crystals are molten and discharged as a melt or mechanically removed.

Preferably solvent is present in the mixture in the crystallizer, although crystallization can also be conducted without solvent. Many solvents for ε-caprolactam are suitable. Examples of suitable solvents are water, alkanes (like n-hexane, n-heptane, iso-octane, cyclohexane), alcohols (like methanol, ethanol, n-propanol, n-butanol), aromatic hydrocarbons (like benzene, toluene, o-xylene, m-xylene, p-xylene), ammonia, chlorinated hydrocarbons (like tetrachloromethane, chloroform or ethyl chloride), ketones (like acetone or methyl ethyl ketone) and esters (like ethyl acetate). Preferably water and aromatic hydrocarbons are used as solvent, since these solvents give large crystals. Most preferred as solvent is water. The solvent will act as a freezing point depressor for the melt in the crystallizer.

In general, melt crystallization requires less energy than solution crystallization, however operations on an industrial scale can be more challenging.

Melt crystallization as used herein in particular means layer melt crystallization or suspension melt crystallization. These two types of technical processes for melt crystallization are characterized by (1) formation of a crystal layer on a heat exchanger wall (layer melt crystallization) and (2) crystals that grow in suspension (suspension melt crystallization). In general, the operation of a process for crystal layer growth on the wall of a heat exchanger is called layer melt crystallization. First, the melt is charged into the crystallizer, then a crystal layer grows on the cooled heat exchanger surface, next the remaining melt that contains impurities that were rejected from the growing crystals, is drained off from the crystallizer and thereafter the crystal layer is melted and the purified product is recovered. The purification efficiency can be further improved by e.g., sweating a.k.a. partial melting, i.e., mildly heating up the crystal layer to nearly its melting temperature causing draining off of entrapped and adherent impure mother liquor. Layer melt crystallization processes are operated in a batchwise mode. Well-known examples of processes that are based on layer melt crystallization are the ProABD process by BEFS Prokem and the Sulzer Chemtech process.

Layer melt crystallization can be performed either in a static or a dynamic mode. In the static crystallization mode, crystals are grown onto the cooling surface from a stagnant melt. At the static mode, the desired compound is crystallized batchwise on a heat exchanger wall from a stagnant melt in a closed vessel. This type of crystallization is characterized by low growth rates of the crystals and as a consequence of that long residence (or batch) times. Preferably, the crystallization time ranges from 1 hour to 75 hours, more preferably, from 2 hours to 50 hours, most preferably from 4 hours to 24 hours. After the crystallization step, the remaining melt is drained off. Then, optionally a sweating phase in introduced to remove impurities that are either adhered to the crystals or are entrapped in the crystals. Finally, the crystals are melted completely and drained off or are mechanically removed.

In general, dynamic crystallization is performed in a tube-and-shell heat exchanger, whereby the melt is circulated downward the cooling surface where the compound crystallizes. In general, the melt is pumped through the tubes and the crystals grow on the inside of the tubes, while the cooling medium is flowing on the outside of the tubes. The thickness of the crystal layer increases in time. After a certain period of time the circulation of melt is stopped and the remaining melt is drained off. Dynamic layer crystallization is like stagnant layer crystallization also performed in a batchwise mode. The crystal growth rates are higher in the dynamic mode compared to the stagnant mode and as a consequence the crystallization times are shorter. Preferably, the crystallization time ranges from 0.05 hour to 12 hours, more preferably, from 0.1 hour to 6 hours, most preferably from 0.3 hours to 3 hours. Then, optionally a sweating phase is introduced to remove impurities that are either adhered to the crystals or are entrapped in the crystals. Finally, the crystals are melted completely and drained off or are mechanically removed.

The suspension melt crystallization can be performed either in a batch or in a continuous mode. With suspension melt crystallization, the melt is cooled below its saturation temperature and crystals start to grow (optionally, after addition of nuclei). The growth rate of the crystals is controlled by the supersaturation temperature of the melt. Suspension melt crystallization can be performed in any exchanger type or vessel type crystallizer that allows cooling of the melt. Preferably, suspension melt crystallization is performed in a scraped surface crystallizer. Optionally, after the crystallization, the resulting mixture of crystals and mother liquor is separated by filtration. Optionally, after the crystallization, the resulting mixture of crystals of the desired compound and mother liquor is charged to a so-called wash column. In a wash column the mother liquor is filtered or drained from the crystals, whereafter, in general, the crystals are washed with purified compound material.

After an ε-caprolactam crystallization step a mother liquor is obtained that comprises next to impurities still ε-caprolactam. Methods to recover ε-caprolactam from this type of mother liquors are well known to the person skilled in the art. And as a result of these recovery methods almost all ε-caprolactam present in the mother liquors can be recovered and converted into high purity ε-caprolactam. One possible solution, in case of multi-stage crystallization, is to recycle mother liquors in a counter-current manner, i.e., mother liquor obtained in the $n^{th}$ crystallization stage is charged to the feed of the $(n-1)^{th}$ crystallization stage. In general, mother liquor obtained from the $1^{rst}$ crystallization stage is charged to an upstream (purification) unit of the process or to a dedicated mother liquor processing unit (e.g., based on distillation or crystallization). After ε-caprolactam crystallization, it may be necessary to purify the obtained mother liquor (or part of it) by, for example, recycling it to any stage prior in the process. Alternatively, mother liquors can be purified, for example, by means of distillation, before being recharged to an ε-caprolactam crystallization step.

The high purity ε-caprolactam obtained according to the process of the invention can be used to make nylon 6 using processes well-known to the skilled person. This nylon 6 may then be used in all known materials, including engineering materials, fibers and films. This nylon 6 that is produced from nylon 6 and polyether polyurethane comprising material according to the invention is especially suitable for high speed spinning applications, including garments containing polyether polyurethane (also known as elastane).

The plant

The invention also provides a plant, i.e., a chemical plant, comprising a separation section [B], a depolymerization section [C], a recovery section [D], and a purification section [E], which is configured to carry out the above-described process of the invention. All plant features specifically described in connection with the process above also apply to the plant of the invention described herein below and vice versa. Thus, the plant is suitable for carrying out the process of the invention and it is to be understood that what has been described in connection with the process of the invention equally applies to the plant embodiments.
The plant can be a laboratory setup as in the examples. Preferably, however, the plant is an industrial scale plant. "Industrial scale" means that the plant has a production capacity for ε-caprolactam (i.e., is in principle capable of producing the same in an amount of) of at least 500 tons per year if operated all the time.
The plant of the invention is suitable for the production of purified ε-caprolactam from nylon 6 and polyether polyurethane comprising material and comprises at least the following four sections: a separation section [B], a depolymerization section [C], a recovery section [D], and a purification section [E]. These sections, and thereby the plant, is configured to carry out the process of the invention described above.
Optionally, the plant of the invention can comprise a pre-treatment section [A], which can comprise a mechanical size reduction section [A] to fragment the nylon 6 and polyether polyurethane comprising material into pieces and/or a cleaning section [ω] to wash the nylon 6 and polyether polyurethane comprising material or the fragmented pieces obtained therefrom. Cleaning includes both washing and separation of foreign materials from the nylon 6 and polyether polyurethane comprising material. The separation of foreign materials can be done both manually (handpicking) and mechanically (e.g., density separation, and magnetic separation). Both manual and mechanical devices, like brushes, can help with the washing in the cleaning section [ω]. The washing is preferably carried out by an additional effect of friction. Different types of industrial washing systems are available on the market, like rotary plastic washers and (high speed) friction washers. The mechanical size reduction section [λ] comprises equipment for the mechanical fragmentation of the nylon 6 and polyether polyurethane comprising material into pieces. Non-limiting examples of this fragmentation equipment are a cutter, a puncher, a shredder, a mill, a grinder, or a chipper.
The separation section [B] can comprise the following four sections: a dissolution section [α], a washing section [β], a precipitation section [γ], and a solvent distillation section [δ]. These sections, and thereby the plant, is configured to carry out the process of the invention described above.
The dissolution section [α] comprises one or more dissolution units that are operated in series and/or in parallel, and one or more separation units that are operated in series and/or in parallel. The optionally cleaned and/or fragmented pieces of nylon 6 and polyether polyurethane comprising material are fed to the dissolution unit(s) as a solid or as a melt, preferably as a solid. The optionally cleaned and/or fragmented pieces of nylon 6 and polyether polyurethane comprising material are optionally dried prior to being fed to the dissolution section [α]. The dissolution unit(s) is/are equipped with inlets for charging separated organic solvent and fresh organic solvent. The dissolution unit(s) is/are equipped with one or more outlets for discharging the mixture obtained in the dissolution unit(s). This mixture comprises non-dissolved nylon 6 and organic solvent in which polyether polyurethane is dissolved. Preferably, the dissolution unit(s) is/are closed. This has the advantage that the emission of organic solvent to the environment is reduced. Preferably, the dissolution unit(s) is/are equipped with means to control the temperature in the unit(s) (e.g., steam coils, steam tracing, electrical tracing). Optionally, the temperature of the separated organic solvent and fresh organic solvent is adjusted (e.g., in a heat exchanger) before being charged to the dissolution section [α].
Good contact between organic solvent and the nylon 6 and polyether polyurethane comprising material is essential for an effective operation. Such contact may be achieved by various means known generally in the art. Improved contact can be achieved by mechanical friction. Mechanical friction in turn can be achieved, e.g., by agitation with a combination of rotating paddles and static fins.
Dissolution units suitable for the dissolution of a polymer in an organic solvent are known to the man skilled in the art. A stirred vessel is an example of such a dissolution unit.
In the separation unit(s) the mixture that is discharged from the dissolution unit(s) is separated into a polyether polyurethane rich stream comprising organic solvent and dissolved polyether polyurethane, and a stream comprising non-dissolved nylon 6. The polyether polyurethane rich stream comprising organic solvent and dissolved polyether polyurethane is discharged via a discharge line and charged to precipitation section [γ]. The stream comprising non-dissolved nylon 6 is discharged via a discharge line and charged to washing section [β], It is advantageous that the

amount of organic solvent in the stream comprising non-dissolved nylon 6 is low in order to facilitate the washing in the washing section [β].

Separation unit(s) suitable for the separation of (non-dissolved) polymer and an organic solvent are known to the man skilled in the art. A centrifuge and a filter are examples of such a separation unit.

Preferably, a dissolution unit and a separation unit are combined in one piece of equipment. This leads to a process intensification.

The washing section [β] comprises equipment for washing the stream comprising non-dissolved nylon 6 with a third solvent to obtain a nylon 6 rich stream and a mixture comprising organic solvent and third solvent. The purpose of the washing section [β] is to remove the organic solvent to a large extent. Optionally, the washing section [β] comprises equipment for drying the nylon 6 rich stream. The mixture comprising organic solvent and third solvent is discharged from the washing section [β] and is charged to the solvent distillation section [δ]. Herein, "charged to the solvent distillation section [δ]" also includes indirect means, i.e., additional steps/sections in between. Preferably, the mixture comprising organic solvent and third solvent that is discharged from the washing section [β] is completely or partly charged to the precipitation section [γ] before being charged to the solvent distillation section [δ].

Optionally after being dried, the nylon 6 rich stream is discharged from the washing section [β] and is charged to the depolymerization section [C].

Good contact between the third solvent and the nylon 6 rich stream is essential for an effective operation. Such contact can be achieved by various means known to the skilled person. One means is a (continuous) centrifuge that is fed with the nylon 6 rich stream and to which the third solvent is charged as washing solvent.

The distillation section [δ] comprises one or more distillation columns that are operated in series and/or in parallel. Distillation columns can be operated in a batch-wise, a semi-continuous or in a continuous mode. The choice between these modes of operation is very much dependent on the scale of operation. In general, batch-wise operation of distillation columns is more suited for treating low volume feed streams. Continuous operation of distillation columns is more suited for treating large volume feed streams.

The exact lay-out of the distillation section [δ] also depends on the nature of the organic solvent, the second solvent and the third solvent.

The distillation section [δ] is charged with the mixture comprising organic solvent and second solvent from which the precipitated polyether polyurethane has been recovered and the mixture comprising organic solvent and third solvent that was discharged from the washing section [β]. Separated organic solvent is discharged from the distillation section [δ] and is charged to the dissolution section [α]. The second solvent and the third solvent are discharged from the distillation section [δ] and are optionally charged to precipitation section [γ] and to the washing section [β], respectively. Residues are discharged from the distillation section [δ]. Optionally, the residues are incinerated, whereby energy is recovered. Finally, degradation products of the used solvents are discharged from the distillation section [δ]. Acetic acid and dimethylamine are examples of degradation products of the organic solvent DMAc.

The depolymerization section [C] comprises one or more depolymerization reactors that are operated in series and/or in parallel. The nylon 6 rich stream is fed to the reactor as a solid or as a melt, preferably as a melt. This feeding may be achieved by using an extruder, gear pump, or other means known in the art.

During production, a depolymerization reactor is at least partially filled with nylon 6-containing feedstock, residual material, ε-caprolactam (and optionally catalyst). The depolymerization reactor can have any desirable form. Preferred reactor types are stirred and non-stirred bubble column reactors, stirred reactors and extruder type reactors.

The depolymerization reactor must be equipped with facilities for feeding of the nylon 6 rich stream, optionally the superheated steam and optionally the catalyst. In addition, the depolymerization reactor is equipped with facilities for discharging the stream comprising ε-caprolactam, and the residual material.

Good contact between the steam and the reactor content is essential for an effective operation. Such contact can be achieved by various means known to the skilled person. As an example, steam can be sparged through the material using a multiplicity of inlets, e.g., using a steam distributor. Further improved contact can be achieved by including mechanical agitation in the reactor, for example, using a combination of rotating paddles and static fins.

Preferably, depolymerization will be complete in 0.5 to 6 hours.

If superheated steam at high temperatures is not available on a production site, it must be made on-purpose by superheating of available steam from a boiler in a so-called superheater.

The recovery section [D] can comprise one or more (preferably partial) condensers to which a ε-caprolactam comprising stream in the form of a vapor stream comprising ε-caprolactam and water is charged. Such a (partial) condenser can have any desirable form. Preferably, a condenser is a distillation column from which a water-rich phase is obtained as top-product and crude ε-caprolactam as bottom product.

The purification section [E] can comprise one or more pieces of extraction equipment, one or more pieces of solvent switch equipment, an oxidation section, a hydrogenation section, one or more pieces of distillation equipment and a crystallization section, to which crude ε-caprolactam is charged and high purity ε-caprolactam is discharged.

To the extraction equipment crude ε-caprolactam and the organic solvent are charged and an organic phase

comprising the organic solvent, ε-caprolactam and impurities, and an aqueous phase comprising water and impurities are discharged. The extraction equipment is selected from mixer-settler extractors, extraction columns, centrifugal extractors, and combinations thereof. Preferably, extraction equipment is a static or an agitated extraction column, like KARR® columns, SCHEIBEL® columns, rotating disc contactors (RDC), pulsed columns, sieve trays (static) columns, random packing (static) columns, and structured packing (SMVP) (static) columns.

To the solvent switch equipment water and an organic phase comprising the organic solvent, ε-caprolactam and impurities are charged and a solvent phase that comprises impurities and an ε-caprolactam-water phase comprising water, ε-caprolactam, and impurities are discharged. The solvent switch equipment for processes based on back-extraction is selected from mixer-settler extractors, extraction columns, centrifugal extractors, and combinations thereof. Preferably, equipment for back-extraction is a static or an agitated extraction column, like KARR® columns, SCHEIBEL® columns, rotating disc contactors (RDC), pulsed columns, sieve trays (static) columns, random packing (static) columns, and structured packing (static) columns.

The solvent switch equipment for processes based on solvent swap distillation is selected from sieve trays distillation columns, random packing distillation columns, and structured packing distillation columns. Preferably, the distillation column is equipped with a reboiler, a condenser and equipment for reflux. The distillation column can be operated at atmospheric pressure, sub-atmospheric pressure or super-atmospheric pressure. Preferably, water is charged to the upper part of the distillation column and the aqueous phase comprising water, ε-caprolactam and impurities with lower- or higher-boiling points than ε-caprolactam is discharged from the lower part of the distillation column.

The optionally present oxidation section comprises one or more oxidation reactors that are operated in series and/or in parallel. An oxidant and the ε-caprolactam-water phase comprising water, ε-caprolactam and impurities are charged to the oxidation section. Usually, the oxidant is charged as solid, as a slurry or as an aqueous solution. In case potassium or sodium permanganate is applied as oxidant, the oxidation section also comprises a filtration section. The oxidation reactor can have any desirable form. Preferred reactor types are stirred and non-stirred reactors and packed column type reactors. The oxidation reactor must be equipped with facilities for feeding of the aqueous phase comprising water, ε-caprolactam and impurities with lower- or higher-boiling points than ε-caprolactam, and the oxidant. In addition, the oxidation reactor must be equipped with facilities for discharging the oxidized ε-caprolactam-water phase comprising water, ε-caprolactam and impurities, and optionally formed solid manganese(IV) oxide ($MnO_2$) particles. Preferably, the oxidation is performed at a temperature ranging from 20 °C to 85 °C and at atmospheric conditions.

The optionally present solid manganese(IV) oxide ($MnO_2$) particles can be removed by settling or by solid-liquid filtration, preferably by solid-liquid filtration. The usage of filter aids, like activated carbon particles or kieselguhr, to improve the filtration procedure are common practice. Filter systems suitable for the separation of solid manganese(IV) oxide particles are known to the skilled person. To such a filter system a suspension of the oxidized ε-caprolactam-water phase comprising the water, ε-caprolactam and impurities, and the solid manganese(IV) oxide particles are charged and the filtered oxidized ε-caprolactam-water phase comprising the water, ε-caprolactam and impurities is discharged. Generally, the solid manganese(IV) oxide particles are retained in the filter system. Preferably, such a filter system is operated in a semi-continuous mode, whereby the suspension and the filtered phase are continuously charged and continuously discharged, while the separated solids are collected in the filter system. From time to time, the charging of the suspension is interrupted and the collected solids are removed from the filter system.

The purification of the crude ε-caprolactam to obtain purified ε-caprolactam in step c.4) optionally comprises a hydrogenation with a heterogeneous catalyst, in which case the plant will comprise a hydrogenation section. Preferably, the catalyst comprises nickel or palladium.

The hydrogenation section comprises one or more hydrogenation reactors that are operated in series and/or in parallel. The hydrogenation can be performed in a three-phase system (gas, liquid, solid) that comprises an aqueous ε-caprolactam mixture, gaseous hydrogen and heterogeneous hydrogenation catalyst. Alternatively, the hydrogenation can be performed in a two-phase system (liquid, solid) that comprises an aqueous ε-caprolactam mixture, that is completely or partially saturated with hydrogen, and heterogeneous hydrogenation catalyst. Dissolution of the hydrogen in the water-ε-caprolactam mixture can be effected by any process that is known to one skilled in the art. Preferably, the mixture is contacted with hydrogen in an absorber or in a mixer in which a constant hydrogen pressure is maintained. Intensive contact between the hydrogen and the mixture will ensure that the hydrogen dissolves in the mixture. Such a process is preferably carried out continuously. The hydrogen-containing mixture is subsequently contacted with the hydrogenation catalyst for example in a separate reactor.

The heterogeneous catalyst can be contacted with the hydrogen-containing reaction mixture in various ways. Hydrogenation may for instance take place in a stirred tank reactor in which the catalyst particles are suspended in the mixture to be hydrogenated (slurry phase process). In such a slurry phase process the catalyst particles and the purified mixture must be separated in an additional process step after the hydrogenation reaction, for instance by means of filtration. Preferably, the catalyst comprises palladium or nickel.

Alternatively, the hydrogenation can be effected in a fixed-bed reactor with the catalyst being fixed in the reactor, so that the additional step for separation of the catalyst and reaction mixture can be dispensed with. Preferably, the fixed

bed is consisting of a supported palladium or nickel catalyst.

The hydrogenation temperature is generally between 20 °C and 160 °C. The hydrogenation pressure is generally between 0.1 and 15 MPa.

To the distillation equipment the ε-caprolactam-water phase comprising water, ε-caprolactam and impurities, that is optionally stripped and/or concentrated and/or optionally oxidized and/or hydrogenated is charged and high purity ε-caprolactam, water, and impurities (i.e., low-boiling organic impurities (having a lower boiling point than ε-caprolactam) and organic high-boiling impurities (having a higher boiling point than ε-caprolactam)) are discharged. The distillation equipment is selected from sieve trays distillation columns, random packing distillation columns, structured packing distillation columns, and horizontal and vertical (falling and climbing) film evaporators. Preferably, the distillation columns are equipped with a reboiler, a condenser, and equipment for reflux. The distillation equipment can be operated at atmospheric pressure, sub-atmospheric pressure or super-atmospheric pressure, preferably at sub-atmospheric pressure.

Preferably, the distillation includes the separation of water, low-boiling organic impurities (having a lower boiling point than ε-caprolactam) and/or organic high-boiling impurities (having a higher boiling point than ε-caprolactam) from ε-caprolactam. Preferably, the distillation includes, in a first step, the separating of water as a top product and the production of ε-caprolactam containing low-boiling impurities and high-boiling impurities as a bottom product. In a second step, low-boiling impurities are separated out as top product and ε-caprolactam containing high-boiling impurities is obtained as a bottom product. In a third step, high purity ε-caprolactam is separated out as a top product and as a bottom product a distillation residue comprising ε-caprolactam and high boiling impurities is produced. Optionally, the first step and the second step are combined.

Preferably, prior to the distillative removal of water and impurities, alkali metal hydroxide, preferably NaOH, is added to the oxidized ε-caprolactam-water phase comprising water, ε-caprolactam and impurities. Preferably, the amount of NaOH that is added ranges from 0.5 to 100 mmol per kg ε-caprolactam, and more preferably from 2 to 80 mmol per kg ε-caprolactam. This leads to a particularly effective distillative removal of impurities with lower and higher boiling points than ε-caprolactam in the subsequent distillation.

The crystallization section comprises one or more crystallizers that are operated in series and/or in parallel. In general, a crystallization section also comprises several vessels to store (intermediate) product streams and/or fresh and used washing liquids. Crystallization of ε-caprolactam can be done by either solution crystallization or melt crystallization, as described before.

In case of solution crystallization ε-caprolactam is recovered by evaporative crystallization, whereby the solvent is evaporated, or by cooling crystallization, whereby the cooling is obtained by direct cooling, indirect cooling or vacuum cooling, or by a combination of these methods. After the crystallization step in a crystallizer, the formed crystals and the mother liquor are separated by e.g., sedimentation in a settler, filtration in a filter and/or centrifugation in a centrifuge. Optionally, the crystallizer is equipped with an agitator and/or one or more baffles.

Optionally, the resulting crystals are washed with e.g., clean solvent. Optionally, the crystallization - separation sequence is repeated several times. The product is obtained as crystals.

The auxiliary solvent is water or a non-aqueous solvent. Examples of non-aqueous solvents include alkanes (like n-hexane, n-heptane, iso-octane, cyclohexane), alcohols (like methanol, ethanol, n-propanol, n-butanol), aromatic hydrocarbons (like benzene, toluene, o-xylene, m-xylene, p-xylene), ammonia, chlorinated hydrocarbons (like tetrachloromethane, chloroform or ethyl chloride), ketones (like acetone or methyl ethyl ketone) and esters (like ethyl acetate), and mixtures of these solvents. In general the auxiliary solvent is recovered and re-used in the crystallization process.

Solution crystallization is usually performed under atmospheric pressure, but may be performed under reduced pressure or under pressured conditions.

Melt crystallization of ε-caprolactam can be done by either layer melt crystallization, whereby an ε-caprolactam-containing crystal layer is formed on a heat exchanger wall, or by suspension melt crystallization, whereby ε-caprolactam-containing crystals grow in suspension.

Preferably solvent is present in the mixture in the melt crystallizer, although melt crystallization can also be conducted without solvent. Many solvents for ε-caprolactam are suitable. Examples of suitable solvents are water, alkanes (like n-hexane, n-heptane, iso-octane, cyclohexane), alcohols (like methanol, ethanol, n-propanol, n-butanol), aromatic hydrocarbons (like benzene, toluene, o-xylene, m-xylene, p-xylene), ammonia, chlorinated hydrocarbons (like tetrachloromethane, chloroform or ethyl chloride), ketones (like acetone or methyl ethyl ketone) and esters (like ethyl acetate). Preferably water and aromatic hydrocarbons are used as solvent, since these solvents give large crystals. Most preferred as solvent is water. The solvent will act as a freezing point depressor for the melt in the crystallizer.

<u>Layer melt crystallization:</u>

First, the melt is charged into the crystallizer, then a crystal layer grows on the cooled heat exchanger surface, next the remaining melt that contains impurities, that were rejected from the growing crystals, is drained off from the

crystallizer and thereafter the crystal layer is melted and the purified product is recovered. The purification efficiency can be further improved by e.g., sweating a.k.a. partial melting, i.e., mildly heating up the crystal layer to nearly its melting temperature causing draining off of entrapped and adherent impure mother liquor. Layer melt crystallization processes are operated in a batchwise mode. Well-known examples of processes that are based on layer melt crystallization are the ProABD process by BEFS Prokem and the Sulzer Chemtech process.

Layer melt crystallization can be performed either in a static or a dynamic mode. In the static crystallization mode, crystals are grown onto the cooling surface from a stagnant melt. At the static mode, the desired compound is crystallized batchwise on a heat exchanger wall from a stagnant melt in a closed vessel. This type of crystallization is characterized by low growth rates of the crystals resulting in long residence (or batch) times. Preferably, the crystallization time ranges from 1 hour to 75 hours, more preferably, from 2 hours to 50 hours, most preferably from 4 hours to 24 hours. After the crystallization step, the remaining melt is drained off. Then, optionally a sweating phase is introduced to remove impurities that are either adhered to the crystals or are entrapped in the crystals. Finally, the crystals are melted completely and drained off or are mechanically removed.

Suspension melt crystallization:

Suspension melt crystallization of ε-caprolactam can be performed either in a batch or in a continuous mode. With suspension melt crystallization, the melt is cooled below its saturation temperature and ε-caprolactam crystals start to grow (optionally, after addition of nuclei). The growth rate of the crystals is controlled by the supersaturation temperature of the melt. Suspension melt crystallization can be performed in any exchanger type or vessel type crystallizer that allows cooling of the melt. Preferably, suspension melt crystallization is performed in a scraped surface crystallizer. Optionally, after the crystallization, the resulting mixture of crystals and mother liquor is separated by filtration. Optionally, after the crystallization, the resulting mixture of ε-caprolactam crystals and mother liquor is charged to a so-called wash column. In a wash column the mother liquor is drained from the ε-caprolactam crystals, whereafter optionally the ε-caprolactam crystals are washed with purified ε-caprolactam.

The process of the invention can be operated in a continuous, semi-continuous or batch-wise fashion. Accordingly, also the plant of the invention can be configured to allow for one or more of these operating modes. In a preferred embodiment, the plant is configured to operate the process of the invention in a continuous or semi-continuous fashion. However, a discontinuous process is also possible. For example, the plant of the invention does not need to contain all sections described herein in one location. In particular, the pre-treatment section [A] can be located at a first location, while the separation section [B], the depolymerization section [C], the recovery section [D] and the purification section [E] are located at a second location. Similarly, also a mechanical size reduction section [λ], as part of the pre-treatment section [A], can be located at a first location, while a cleaning section [ω], as part of pre-treatment section [A] can be located at a second location, while the separation section [B], the depolymerization section [C], the recovery section [D] and the purification section [E] are located at a third location. Optionally, the cleaning section [ω] is split in two or more segments that are optionally all located at different locations. E.g., a first segment of cleaning section [ω], as part of pre-treatment section [A] can be located at a first location, a mechanical size reduction section [λ], as part of the pre-treatment section [A], can be located at a second location, while a second segment of cleaning section [ω], as part of pre-treatment section [A] can be located at a third location, while the separation section [B], the depolymerization section [C], the recovery section [D] and the purification section [E] are located at a fourth location. Optionally, the cleaning section [ω], as part of pre-treatment section [A] is split in two or more segments that are optionally all located at different locations. Optionally, the cleaning section [ω], as part of pre-treatment section [A] is located at the same location than separation section [B]. Optionally, the separation section [B], the depolymerization section [C] and the recovery section [D] are located at a different location than pre-treatment section [A] and the purification section [E]. Optionally, the depolymerization section [C] and the recovery section [D] are located at a different location than pre-treatment section [A] and the separation section [B].

The product

The invention provides as new products ε-caprolactam and polyether polyurethane both obtained via depolymerization of nylon 6 and polyether polyurethane comprising material according to the process of the invention, which meet the specification for high demanding applications, and at the same time the process is particularly environmentally friendly due to its lowered product carbon footprint and the use of waste as starting material. This ε-caprolactam obtained by the process of the invention is advantageously characterized in particular by having a product carbon footprint of less than 3 kg $CO_2$ per kg purified ε-caprolactam. The ε-caprolactam obtained according to the invention may also be referred to as "purified ε-caprolactam". "Purified" as used herein means that the ε-caprolactam is produced from nylon 6 and polyether polyurethane comprising material according to the process of the invention, which results in it being obtained in purified form. In this sense, the ε-caprolactam is obtained and purified from nylon 6 and polyether polyurethane

comprising material.

The process of the invention allows producing high-purity and therefore high-quality ε-caprolactam, which meets the specification for high demanding applications and at the same time the process is particularly economically friendly due to its lowered product carbon footprint and the use of waste as starting material. In preferred embodiments, the ε-caprolactam obtained by the process of the invention fulfils one or more of the following specifications, wherein the parameters and measurement methods are defined as in the Example section herein below:

| | |
|---|---|
| PAN: | max. 5 |
| E290: | max. 0.05 |
| VB: | max. 0.5 mmol/kg |
| Alkalinity: | max. 0.1 mmol/kg |
| Acidity: | max. 0.1 mmol/kg |

The ε-caprolactam produced by the process of the invention is also particularly economical and environmentally friendly. This is evident from the much lower carbon footprint of the ε-caprolactam produced by the process of the invention as compared to classically produced ε-caprolactam (e.g., by Beckmann rearrangement of cyclohexanone oxime).

The environmental impact of a product is generally expressed as 'product carbon footprint'. The carbon footprint of a product is defined as the total emissions caused by the formation of that product, expressed as ton carbon dioxide equivalent per ton product. The carbon footprint of a product is amongst others depending on the feedstock, auxiliary materials, energy consumption, energy sources, production process and process efficiencies. Quantification of the carbon footprint of a product can be done as described in, e.g., the European Standard EN ISO 14040:2006 ("Environmental management - Life cycle assessment - Principles and framework).

Product carbon footprint calculations can be done both in-house or by external (preferably) certified organizations. These organizations verify and certify the product carbon footprint calculations based on e.g., LCA standard ISO 14040.

J. Hong and X. Xu ("Environmental impact assessment of caprolactam production - a case study in China"; J. of Cleaner production 27 (2012) 103-108; DOI: 10.1016/j.jclepro.2011.12.037) reported that the potential impact of "virgin" ε-caprolactam obtained via Beckmann rearrangement of cyclohexanone oxime on global warming is 7.5 ton $CO_2$ equivalent per ton ε-caprolactam (which is equal to 7.5 kg $CO_2$ equivalent per kg ε-caprolactam), in case coal-based electricity and steam generation are involved. If natural gas-based electricity and steam generation are involved, the potential impact of virgin ε-caprolactam on global warming of the ε-caprolactam production process will be reduced to 6.4 ton $CO_2$ equivalent per ton ε-caprolactam (which is equal to 6.4 kg $CO_2$ equivalent per kg ε-caprolactam).

The product carbon footprint of ε-caprolactam that is obtained according to the process of the invention is much lower than the one of *de novo* synthesized, or "virgin" ε-caprolactam. The product carbon footprint of the ε-caprolactam that is obtained in the process of the invention is less than 4 kg, more preferably less than 3 kg, and most preferably equal to or less than 3.0 kg $CO_2$ equivalent per kg ε-caprolactam.

The polyether polyurethane produced by the process of the invention is also particularly economically and environmentally friendly. Again, this is evident from the much lower carbon footprint of the polyether polyurethane produced by the process of the invention as compared to classically produced polyether polyurethane, e.g., by reaction of polyether polyols and diisocyanate monomer.

N.M. van der Velden, M.K. Patel and J.G. Vogtländer (Table 7 in: "LCA benchmarking study on textiles made of cotton, polyester, nylon, acryl, or elastane", Int J Life Cycle Assess (2014) 19:331-356; DOI: 10.1007/s11367-013-0626-9) reported that the potential impact of virgin elastane production on global warming is equal to 4.836 kg $CO_2$ equivalent per kg elastane.

The product carbon footprint of polyether polyurethane that is obtained according to the process of the invention is much lower than the one of *de novo* synthesized, or "virgin" polyether polyurethane. The product carbon footprint of the polyether polyurethane that is obtained according to the process of the invention is less than 3.0 kg, preferably less than 2.0 kg $CO_2$, and most preferably less than 1.0 kg $CO_2$ equivalent per kg polyether polyurethane.

## BRIEF DESCRIPTION OF THE DRAWINGS

In the following, the invention is described with reference to the Figures, which depict certain embodiments of the invention. The invention, however, is as defined in the claims and as generally described herein. It should not be limited to the embodiments shown for illustrative purposes in the Figures below.

FIG. 1 is a schematic of the process of the invention for the recovery of both purified ε-caprolactam and polyether

polyurethane from nylon 6 and polyether polyurethane comprising material, comprising processing steps performed in an optional pre-treatment section [A], a separation section [B], a depolymerization section [C], a recovery section [D] and a purification section [E].

FIG. 2 illustrates two embodiments of the pre-treatment section [A], in which the nylon 6 and polyether polyurethane comprising material is cleaned in a cleaning section [ω] by removal of foreign materials and by washing with a washing solvent and fragmented in a mechanical size reduction section [A] to obtain cleaned and fragmented pieces of nylon 6 and polyether polyurethane comprising material.

FIG. 2A depicts an embodiment of the pre-treatment section [A], in which the nylon 6 and polyether polyurethane comprising material is first cleaned in a cleaning section [ω] by removal of foreign materials and by washing with a washing solvent and then fragmented in a mechanical size reduction section [A] to obtain cleaned and fragmented pieces of nylon 6 and polyether polyurethane comprising material.

FIG. 2B depicts an embodiment of the pre-treatment section [A], in which nylon 6 and polyether polyurethane comprising material is first fragmented in a mechanical size reduction section [A] and then cleaned in a cleaning section [ω] by removal of foreign materials and by washing with a solvent to obtain cleaned and fragmented pieces of nylon 6 and polyether polyurethane comprising material.

FIG. 3 illustrates an embodiment of the separation section [B], in which the nylon 6 and polyether polyurethane comprising material is separated to obtain a nylon 6 rich stream and recovered polyether polyurethane. The plant used in this embodiment comprises a dissolution section [α], a washing section [β], a precipitation section [γ], and a solvent distillation section [δ].

FIG. 4 illustrates two embodiments of the purification section [E], in which crude ε-caprolactam is purified to obtain high purity ε-caprolactam.

FIG. 4A depicts an embodiment of the purification section [E] of the process of the invention, that comprises an extraction section [K], optionally a washing section [L], optionally a back-extraction section [M], optionally a stripping and concentration section [N], optionally a distillation section [O], and a crystallization section [P].

FIG. 4B depicts an embodiment of the purification section [E] of the process of the invention, that comprises an extraction section [K], optionally a washing section [L], optionally a solvent swap distillation section [R], optionally a stripping and concentration section [N], optionally a distillation section [O], and a crystallization section [P].

Detailed description of the drawings

The process of the invention is schematically illustrated in FIG. 1. The process is carried out in the following plant sections: optionally a pre-treatment section [A], a separation section [B], a depolymerization section [C], a recovery section [D] and a purification section [E].

In the optional pre-treatment section [A] nylon 6 and polyether polyurethane comprising material [301] is cleaned and/or fragmented by mechanical size reduction to obtain cleaned and/or fragmented pieces of nylon 6 and polyether polyurethane comprising material [304] that is/are discharged. Optionally, in the pre-treatment section [A], in which the nylon 6 and polyether polyurethane comprising material [301] is cleaned by removal of foreign materials and/or by washing with a washing solvent [302], foreign materials and contaminated washing solvent [303] are obtained that are discharged. Removal of foreign materials can be done prior and/or after the washing with a washing solvent [302]. Cleaning can be done prior and/or after the fragmentation of the nylon 6 and polyether polyurethane comprising material [301]. Optionally, the cleaned and/or fragmented pieces of nylon 6 and polyether polyurethane comprising material [304] are dried prior to being charged to the separation section [B]. Optionally, the cleaned and/or fragmented pieces of nylon 6 and polyether polyurethane comprising material are densified before being charged to separation section [B].

In the separation section [B], the optionally cleaned and/or fragmented pieces of nylon 6 and polyether polyurethane comprising material [304] are separated to obtain a nylon 6 rich stream [311] and recovered polyether polyurethane [308], that are discharged from separation section [B]. The nylon 6 rich stream [311] is charged to depolymerization section [C]. Optionally, the nylon 6 rich stream [311] and/or recovered polyether polyurethane [308] are dried before being discharged from separation section [B]. Optionally, nylon 6 rich stream [311] is densified before being depolymerized to ε-caprolactam in the depolymerization section [C]. Separation section [B] comprises the following sections: a dissolution section [α], a washing section [β], a precipitation section [γ], and a solvent distillation section [δ] (not shown in FIG. 1). Organic solvent [305] is charged to separation section [B] to dissolve polyether polyurethane. A second solvent [306] is charged to separation section [B] to precipitate dissolved polyether polyurethane. A third solvent [307] is charged to separation section [B] to wash non-dissolved nylon 6. After distillative separation the second solvent and the third solvent [309] and residues [310] are discharged from separation section [B].

The optionally dried and/or densified nylon 6 rich stream [311] is depolymerized to ε-caprolactam in the depolymerization section [C]. An ε-caprolactam comprising stream [315] is discharged from the depolymerization section [C]. In addition, residual material [314] is discharged. Optionally, superheated steam [312] and catalyst [313] are charged to the depolymerization section [C].

Crude ε-caprolactam [317] is recovered from the ε-caprolactam comprising stream [315] that is discharged from the depolymerization section [C]. The crude ε-caprolactam [317] is discharged from the recovery section [D] and is charged to purification section [E]. In addition, an aqueous phase [316] is discharged from the recovery section [D] in case water or superheated steam [312] is charged to depolymerization section [C] or (not shown in FIG. 1) water is charged to the recovery section [D].

Crude ε-caprolactam [317] that is discharged from recovery section [D] is purified to yield high purity ε-caprolactam [319] in purification section [E]. Water and impurities [318] are also discharged from the purification section [E].

FIG. 2A depicts an embodiment of the pre-treatment section [A] (area enclosed by dashed line), in which nylon 6 and polyether polyurethane comprising material [31] is first cleaned in a cleaning section [ω] by removal of foreign materials and by washing with a washing solvent [32] whereby foreign materials and contaminated washing solvent [33] and cleaned nylon 6 and polyether polyurethane comprising material [34] are obtained. Subsequently, the cleaned nylon 6 and polyether polyurethane comprising material [34] is fragmented in a mechanical size reduction section [A] to obtain cleaned and fragmented pieces of nylon 6 and polyether polyurethane comprising material [35]. The cleaned and fragmented nylon 6 and polyether polyurethane comprising material [35] is then discharged. Optionally, the cleaned and fragmented pieces of nylon 6 and polyether polyurethane comprising material [35] are densified before being separated into a nylon 6 rich stream and recovered polyether polyurethane in the separation section [B] (not shown in FIG. 2A).

FIG. 2B depicts an embodiment of the pre-treatment section [A] (area enclosed by dashed line), in which nylon 6 and polyether polyurethane comprising material [41] is first fragmented in a mechanical size reduction section [A] to obtain fragmented pieces of nylon 6 and polyether polyurethane comprising material [42]. Subsequently, the fragmented pieces of nylon 6 and polyether polyurethane comprising material [42] are cleaned in a cleaning section [ω] by removal of foreign materials and by washing with a washing solvent [43] to obtain foreign materials and contaminated washing solvent [44], and cleaned and fragmented pieces of nylon 6 and polyether polyurethane comprising material [45]. The cleaned and fragmented pieces of nylon 6 and polyether polyurethane comprising material [45] are then discharged. Optionally, the cleaned and fragmented pieces of nylon 6 and polyether polyurethane comprising material [45] are densified before being separated into a nylon 6 rich stream and recovered polyether polyurethane in the separation section [B] (not shown in FIG. 2B).

FIG. 3 illustrates an embodiment of the separation section [B] (area enclosed by the dashed line), in which the nylon 6 and polyether polyurethane comprising material is separated to obtain a nylon 6 rich stream and recovered polyether polyurethane. This embodiment comprises the following sections: a dissolution section [α], a washing section [β], a precipitation section [γ], and a solvent distillation section [δ].

In the dissolution section [α] polyether polyurethane is dissolved in an organic solvent from the optionally cleaned and/or fragmented pieces of polyamide 6 and polyether polyurethane comprising material [101] to obtain a polyether polyurethane rich stream comprising organic solvent and dissolved polyether polyurethane [104] and a stream comprising non-dissolved nylon 6 [105]. These are discharged from dissolution section [α]. The organic solvent is the separated organic solvent [102] to which optionally fresh organic solvent [103] is added.

In the precipitation section [γ] a second solvent [106] and the polyether polyurethane rich stream comprising organic solvent and dissolved polyether polyurethane [104] are mixed so that polyether polyurethane is precipitated from a mixture comprising organic solvent and second solvent. The precipitated polyether polyurethane is recovered (e.g., by filtration or centrifugation) from the mixture comprising organic solvent and second solvent. The precipitated polyether polyurethane that is recovered is discharged as recovered polyether polyurethane [107] from precipitation section [γ]. Optionally, the recovered polyether polyurethane [107] is dried after the recovery step and prior to being discharged from the precipitation section [γ]. The mixture comprising organic solvent and second solvent from which precipitated polyether polyurethane has been recovered [108] is discharged from the precipitation section [γ] and charged to the solvent distillation section [δ].

In the washing section [β] the stream comprising non-dissolved nylon 6 [105] is washed with a third solvent [109] to obtain a nylon 6 rich stream [110] and a mixture comprising organic solvent and third solvent [111]. The nylon 6 rich stream [110] is discharged from the washing section [β]. Optionally, the nylon 6 rich stream [110] is dried after the washing step and prior to being discharged from the washing section [β]. Optionally, the nylon 6 rich stream [110] is densified before being depolymerized to ε-caprolactam in the depolymerization section [C] (not shown in FIG. 3). The mixture comprising organic solvent and third solvent [111] is discharged from the washing section [β] and optionally charged to the solvent distillation section [δ].

In the solvent distillation section [δ] the mixture comprising organic solvent and second solvent from which precipitated polyether polyurethane has been recovered [108] and optionally the mixture comprising organic solvent and third solvent [111] are distilled to obtain separated organic solvent [102], second solvent and third solvent [112], and residues [113]. Optionally, the second solvent and the third solvent are both water, in which case separated organic solvent [102] is dried organic solvent. The separated organic solvent [102] is charged to dissolution section [α].

FIG. 4A depicts an embodiment of the purification section [E] (area enclosed by dashed line), that comprises the following sections:

In the extraction section [K], crude ε-caprolactam [201] is extracted with an organic solvent [202] to obtain an aqueous phase comprising water and impurities [203] and an organic phase comprising the organic solvent, ε-caprolactam and impurities [204]. Both phases are discharged from the extraction section [K].

In the optional washing section [L], the organic phase comprising the organic solvent, ε-caprolactam and impurities [204] is washed with water or an aqueous alkaline solution [205] to obtain an aqueous residue-comprising phase [206] and a washed organic phase that comprises organic solvent, ε-caprolactam and impurities [207]. Both phases are discharged from the washing section [L].

In the optional back-extraction section [M], the optionally washed organic phase that comprises the organic solvent, ε-caprolactam and impurities [207] is back-extracted with water [208] to obtain an organic solvent phase that comprises impurities [209] and an aqueous phase comprising water, ε-caprolactam and impurities with lower- or higher-boiling points than ε-caprolactam [210]. Both phases are discharged from the back-extraction section [M].

In the optional stripping and concentration section [N], residual organic solvent and water are removed by stripping and/or distillation from the aqueous phase comprising water, ε-caprolactam and impurities with lower- or higher-boiling points than ε-caprolactam [210] to obtain residual organic solvent and water [211] and a stripped and concentrated aqueous phase comprising water, ε-caprolactam and impurities with lower- or higher-boiling points than ε-caprolactam [212]. Both phases are discharged from the stripping and concentration section [N].

Optionally, the aqueous phase comprising water, ε-caprolactam and impurities with lower- or higher-boiling points than ε-caprolactam [210], from which optional residual organic solvent and water has been removed by stripping and/or distillation, is oxidized with an oxidant to obtain an oxidized ε-caprolactam-water phase comprising water, ε-caprolactam, and impurities (not shown in FIG. 4A).

Optionally, the oxidized ε-caprolactam-water phase comprising the water, ε-caprolactam and impurities is filtered to remove solid manganese(IV) oxide particles before being charged to the next section (not shown in FIG. 4A).

In the optional hydrogenation section, the stripped and concentrated aqueous phase comprising water, ε-caprolactam and impurities with lower- or higher-boiling points than ε-caprolactam [212] is hydrogenated with hydrogen in the presence of a heterogeneous catalyst to obtain a hydrogenated ε-caprolactam-water phase comprising water, ε-caprolactam, and impurities before being charged to the next section (not shown in FIG. 4A).

In optional distillation section [O], the stripped and concentrated aqueous phase comprising water, ε-caprolactam and impurities with lower- or higher-boiling points than ε-caprolactam [212] is distilled to remove impurities with lower- or higher-boiling points than ε-caprolactam [214] and optionally organic solvent or water (not shown in FIG. 4A) and whereby is obtained a distilled ε-caprolactam phase [215]. All of the distillation products are discharged from the distillation section [O]. The distilled ε-caprolactam phase [215] is charged to the crystallization section [P]. Optionally, prior to the distillation in distillation section [O], an alkali metal hydroxide [213] is dosed to the stripped and concentrated aqueous phase comprising water, ε-caprolactam and impurities with lower- or higher-boiling points than ε-caprolactam [212].

In crystallization section [P], the optionally distilled ε-caprolactam phase [215] is crystallized at a temperature of 10 °C to 95 °C to remove impurities [217] from ε-caprolactam and whereby is obtained high purity ε-caprolactam [218]. All of the crystallized products are discharged from the crystallization section [P]. Optionally, prior to the crystallization a solvent [216] is charged to the crystallization section [P].

FIG. 4B depicts an embodiment of the purification section [E] (area enclosed by dashed line), that comprises the following sections:

In the extraction section [K], crude ε-caprolactam [401] is extracted with an organic solvent [402] to obtain an aqueous phase comprising water and impurities [403] and an organic phase comprising the organic solvent, ε-caprolactam and impurities [404]. Both phases are discharged from the extraction section [K].

In the optional washing section [L], the organic phase comprising the organic solvent, ε-caprolactam and impurities [404] is washed with water or an aqueous alkaline solution [405] to obtain an aqueous residue-comprising phase [406] and a washed organic phase that comprises organic solvent, ε-caprolactam and impurities [407]. Both phases are discharged from the washing section [L].

In the optional solvent swap distillation section [R], the optionally washed organic phase comprising the organic solvent, ε-caprolactam and impurities [407] is solvent swap distilled with addition of water [408] to obtain an organic solvent comprising stream [409] and an aqueous phase comprising water, ε-caprolactam and impurities with lower- or higher-boiling points than ε-caprolactam [410]. Both products are discharged from the solvent swap distillation section [R].

In the optional stripping and concentration section [N], residual organic solvent and water are removed by stripping and/or distillation from the aqueous phase comprising water, ε-caprolactam and impurities with lower- or higher-boiling points than ε-caprolactam [410] to obtain residual organic solvent and water [411] and a stripped and concentrated aqueous phase comprising water, ε-caprolactam and impurities with lower- or higher-boiling points than ε-caprolactam [412]. Both phases are discharged from the stripping and concentration section [N].

Optionally, the aqueous phase comprising water, ε-caprolactam and impurities with lower- or higher-boiling points than ε-caprolactam [410], from which optional residual organic solvent and water has been removed by stripping

and/or distillation, is oxidized with an oxidant to obtain an oxidized ε-caprolactam-water phase comprising water, ε-caprolactam, and impurities (not shown in FIG. 4B).

Optionally, the oxidized ε-caprolactam-water phase comprising the water, ε-caprolactam and impurities is filtered to remove solid manganese(IV) oxide particles before being charged to the next section (not shown in FIG. 4B).

In the optional hydrogenation section, the stripped and concentrated aqueous phase comprising water, ε-caprolactam and impurities with lower- or higher-boiling points than ε-caprolactam [412] is hydrogenated with hydrogen in the presence of a heterogeneous catalyst to obtain a hydrogenated ε-caprolactam-water phase comprising water, ε-caprolactam, and impurities before being charged to the next section (not shown in FIG. 4B).

In optional distillation section [O], the stripped and concentrated aqueous phase comprising water, ε-caprolactam and impurities with lower- or higher-boiling points than ε-caprolactam [412] is distilled to remove impurities with lower- or higher-boiling points than ε-caprolactam [414] and optionally organic solvent or water (not shown in FIG. 4B) and whereby is obtained a distilled ε-caprolactam phase [415]. All of the distillation products are discharged from the distillation section [O]. The distilled ε-caprolactam phase [415] is charged to the crystallization section [P]. Optionally, prior to the distillation in distillation section [O], an alkali metal hydroxide [413] is dosed to the stripped and concentrated aqueous phase comprising water, ε-caprolactam and impurities with lower- or higher-boiling points than ε-caprolactam [412].

In crystallization section [P], the optionally distilled ε-caprolactam phase [415] is crystallized at a temperature of 10 °C to 95 °C to remove impurities [417] from ε-caprolactam and whereby is obtained high purity ε-caprolactam [418]. All of the crystallized products are discharged from the crystallization section [P]. Optionally, prior to the crystallization a solvent [416] is charged to the crystallization section [P].

## Examples

The following examples serve to explain the invention in more detail, in particular with regard to certain forms of the invention. The examples, however, are not intended to limit the present disclosure.

ε-caprolactam, that can be used for all major nylon 6 polymerization applications, without dilution with purer qualities of ε-caprolactam, fulfils all of the following specifications:

| | |
|---|---|
| PAN: | max. 5 |
| E290: | max. 0.05 |
| VB: | max. 0.5 mmol/kg |
| Alkalinity: | max. 0.1 mmol/kg |
| Acidity: | max. 0.1 mmol/kg |

The parameters and measurement methods are defined as follows:

PAN : ISO DIS 8660-Plastics-Determination of permanganate index of caprolactam-Spectrometric method, revision of first edition ISO 8660; 1988,

E290 : ISO 7059-caprolactam for industrial use-determination of absorbance at a wavelength of 290 nm,

Volatile bases (VB) ISO 8661-Caprolactam for industrial use-Determination of volatile bases content-Titrimetric method after distillation.

Alkalinity of ε-caprolactam product: the alkalinity is determined by titration at a temperature of 25 °C using a Tashiro indicator in a 1 : 2 ratio of 0.1 wt./$v_{Ethanol}$% Methylene blue : 0.1 wt./$v_{Ethanol}$% Methyl red, which is grey at its end point. A flask containing water and indicator is first titrated to grey, then X grams of an aqueous ε-caprolactam solution containing Y wt.% ε-caprolactam (as determined by refractive index) is added and the solution is titrated back to grey using a 0.01 N $H_2SO_4$ solution (in case the solution is alkaline) or a 0.01 N NaOH solution (in case the solution is acidic).

Alkalinity is then given by:

$$\text{Alkalinity (mmol/kg } \varepsilon\text{-caprolactam)} = v * t * 1000 / (X * Y)$$

Where:

v = volume of $H_2SO_4$ solution added (ml)

t = normality of $H_2SO_4$ solution (= 0.01 N)
X = weight of sample (g)
Y = concentration ε-caprolactam (wt.%)

Acidity is then given by:

$$\text{Acidity (mmol/kg ε-caprolactam)} = v * t * 1000 / (X * Y)$$

Where:

v = volume of NaOH solution added (ml)
t = molarity of NaOH solution (=0.01 N)
X = weight of sample (g)
Y = concentration ε-caprolactam (wt.%)

EXAMPLE 1

Pre-treatment and separation of nylon 6 and polyether polyurethane.

The polyether polyurethane content of the used polyether polyurethane containing nylon 6 wasted fabric was 20.2 +/- 0.4 wt.% (as determined by Differential Scanning Calorimetry (DSC) Method ISO11357-3_-130 °C to 300 °C_10 °C/min_dt 1.00 s).

The polyether polyurethane containing nylon 6 wasted fabric was cut in small pieces ranging from 5 to 20 $cm^2$ each. 150.4 grams of this cut material was dissolved in 800 grams of DMAc at a temperature of 70 °C for 1 hour under stirring. The resulting solution was vacuum filtered through a heated double walled Buchner funnel at a temperature of 70 °C, whereby filtrate 1 was obtained. The remaining non-dissolved material was further treated three times with each time 400 grams DMAc at a temperature of 70 °C for 1 hour under stirring, whereby the filtrates 2 to 4 were obtained.

The non-dissolved material on the Buchner funnel was washed with 325 grams of water at a temperature of 65 °C, whereby filtrate 5 was obtained and then the non-dissolved material was dried and afterwards weighed. The five filtrates were combined, whereby a precipitate and a clear solution were obtained. The precipitate was filtered off and washed with 200 grams of water and subsequently dried and afterwards weighed. The total weight of the dried precipitate and the dried non-dissolved material was almost equal to the weight of the starting material.

Analysis by Differential Scanning Calorimetry (DSC) revealed that the polyether polyurethane content of the dry precipitate was circa 100 wt.% (polyamide 6 was not detected). Based on DSC analysis, the nylon 6 content of the dry non-dissolved material was circa 100 wt.% (polyether polyurethane was not detected).

The recovered polyether polyurethane precipitate can be re-used as-such or in combination with virgin polyether polyurethane in the production of textiles.

This EXAMPLE shows that polyether polyurethane and nylon 6 can be separated from polyether polyurethane containing nylon 6 wasted fabric by selective extraction, precipitation of the dissolved polyether polyurethane and washing of the non-dissolved nylon 6. After drying, the recovered precipitate consists of almost pure polyether polyurethane and the nylon 6 content of the dry non-dissolved material was almost 100 wt.%.

EXAMPLE 2

Pre-treatment and separation of nylon 6 and polyether polyurethane.

The polyether polyurethane content of the used polyether polyurethane containing nylon 6 wasted fabric was 8.15 +/- 0.05 wt.% (as determined by Differential Scanning Calorimetry (DSC) Method ISO11357-3_-130 °C to 300 °C_10 °C/min_dt 1.00 s).

The procedure of EXAMPLE 1 was followed except that now 254.1 grams of the polyether polyurethane containing nylon 6 wasted fabric was dissolved in 1200 grams of DMAc and after filtration 475 grams of DMAc and 350 grams of water were used for washing of the remaining non-dissolved material on the Buchner funnel. The precipitate was filtered off and washed with 200 grams of water and subsequently dried. The non-dissolved material on the Buchner funnel was washed with 1000 grams of water then also dried and afterwards weighed. The weight fraction of the dry precipitate was 8 wt.% of the total weight of the dry precipitate and the dry non-dissolved material, which was almost equal to the weight of the starting material.

Analysis by Differential Scanning Calorimetry (DSC) revealed that the polyether polyurethane content of the

dry precipitate was circa 100 wt.% (nylon 6 was not detected). The nylon 6 content and the polyether polyurethane content of the dry non-dissolved material were circa 99 wt.% and less than 1 wt.%, respectively (DSC analysis).

The recovered polyether polyurethane precipitate can be re-used as-such or in combination with virgin polyether polyurethane in the production of textiles.

This EXAMPLE shows that polyether polyurethane and nylon 6 can be separated from polyether polyurethane containing nylon 6 wasted fabric by selective extraction, precipitation of the dissolved polyether polyurethane and washing of the non-dissolved nylon 6. After drying, the recovered precipitate consists of almost pure polyether polyurethane and the polyether polyurethane content of the non-dissolved material is much lower than that of the starting material polyether polyurethane containing nylon 6 wasted fabric. The obtained polyether polyurethane is of high quality and can be used as-such or in combination with virgin polyether polyurethane in demanding follow-up applications, including spinning.

EXAMPLE 3

Depolymerization of nylon 6 and recovery of ε-caprolactam.

The dry non-dissolved material obtained in EXAMPLE 1 was first densified before it was charged to the depolymerization reactor. The dry non-dissolved material was melted under nitrogen at 237 °C and forced through a perforated metal plate. The obtained strands were cooled down to room temperature and cut into pellets. The diameter and the length of the resulting pellets were 3 mm and 1 cm, respectively.

33.6 grams of these nylon 6 containing pellets and 9.5 grams of 20 wt.% phosphoric acid were charged to a Premex high pressure autoclave. First, the reactor content was heated under nitrogen and subsequently superheated steam was injected continuously at a rate of 2.7 grams per minute during the 120-minute reaction. The temperature and the pressure in the reactor were maintained at 260 °C and 0.11 MPa, respectively. During the reaction a vapor stream was continuously discharged from the reactor and was cooled down to ca. 20 °C, whereby an ε-caprolactam and water comprising condensate was obtained.

The condensate that composed of 25.7 grams of ε-caprolactam, most of the remainder being water, was concentrated by evaporation in a rotavap (rotary evaporator) that was operated under vacuum (9.5 kPa; water bath temperature was ca. 65 °C) to an ε-caprolactam concentration of 57.3 wt.%. (This mixture, crude ε-caprolactam, is the mixture to be purified.)
The specifications of the crude ε-caprolactam were:

| | |
|---|---|
| PAN: | 353 |
| E290: | 2.91 |

This EXAMPLE shows that crude ε-caprolactam can be obtained in good yield and without operational issues by depolymerization of nylon 6 that has been obtained by extractive separation from polyether polyurethane containing nylon 6 wasted fabric.

EXAMPLE 4

Depolymerization of nylon 6 and recovery of ε-caprolactam.

The procedure of EXAMPLE 3 was followed except that now 48 grams of pellets made from dry non-dissolved material obtained in EXAMPLE 2 and 14 grams of 20 wt.% phosphoric acid were charged to a Premex high pressure autoclave.

The condensate that composed of 41 grams of ε-caprolactam, most of the remainder being water, was concentrated by evaporation in a rotavap (rotary evaporator) that was operated under vacuum (9.5 kPa; water bath temperature was ca. 65 °C) to an ε-caprolactam concentration of 63.1 wt.%. (This mixture, crude ε-caprolactam, is the mixture to be purified.)
The specifications of the crude ε-caprolactam were:

| | |
|---|---|
| PAN: | 274 |
| E290: | 2.70 |

This EXAMPLE shows that crude ε-caprolactam can be obtained in good yield and without operational issues by depolymerization of nylon 6 that has been obtained by extractive separation from polyether polyurethane containing

nylon 6 wasted fabric.

COMPARATIVE EXPERIMENT 1

Depolymerization of nylon 6 and polyether polyurethane.

The procedure of EXAMPLE 3 was followed except that now 37.6 grams of polyether polyurethane fibers (same material as included in the wasted fabrics used in EXAMPLE 1) and 14 grams of 20 wt.% phosphoric acid were charged to a Premex high pressure autoclave.

10 minutes after starting the injection of the superheated steam the experiment had to be stopped, because the line for discharging the ε-caprolactam and water comprising vapor stream was clogged by an insoluble material.

From this COMPARATIVE EXPERIMENT, it can be concluded that depolymerization of polyether polyurethane containing nylon 6 wasted fabric without removal of polyether polyurethane prior to the depolymerization can give operational issues.

COMPARATIVE EXPERIMENT 2

Depolymerization of polyether polyurethane containing nylon 6 wasted fabric and recovery of ε-caprolactam.

The procedure of EXAMPLE 3 was followed except that now 48 grams of pellets made from polyether polyurethane containing nylon 6 wasted fabric with a polyether polyurethane content of 20.2 +/- 0.4 wt.% (same feedstock as used in EXAMPLE 1) and 14 grams of 20 wt.% phosphoric acid were charged to a Premex high pressure autoclave.

The condensate that composed of 26 grams of ε-caprolactam, most of the remainder being water, was concentrated by evaporation in a rotavap that was operated under vacuum (9.5 kPa; water bath temperature was ca. 65 °C) to an ε-caprolactam concentration of 47.0 wt.%. (This mixture, crude ε-caprolactam, is the mixture to be purified.) The specifications of the crude ε-caprolactam were:

| | |
|---|---|
| PAN: | 352 |
| E290: | 3.60 |

Observations:

- The obtained condensate before concentration did contain an unknown precipitate
- Fouling of the internal walls of the Premex high pressure autoclave was seen after this depolymerization experiment

This COMPARATIVE EXPERIMENT again shows that depolymerization of polyether polyurethane containing nylon 6 wasted fabric without removal of polyether polyurethane prior to the depolymerization gives rise to operational issues. Another observation is that the ε-caprolactam yield in this COMPARATIVE EXPERIMENT is much lower than in EXAMPLE 3 (with pre-treated polyether polyurethane containing nylon 6 wasted fabric as feed), whereby the ε-caprolactam yield is defined as the ratio of the weight of ε-caprolactam in the condensate to the weight of nylon 6 in the feed that is charged to the Premex high pressure autoclave.

COMPARATIVE EXPERIMENT 3

Purification by distillation.

75 mmol of aqueous sodium hydroxide per kg ε-caprolactam was then added to the crude ε-caprolactam obtained in COMPARATIVE EXPERIMENT 2. This mixture was then distilled by reducing the pressure in steps. ε-caprolactam was distilled at 300 Pa.
The specifications of the distilled ε-caprolactam were:

| | |
|---|---|
| PAN: | 31 |
| E290: | 3.08 |
| VB: | 2.45 mmol/kg |
| Alkalinity: | 3.13 mmol/kg |

This COMPARATIVE EXPERIMENT shows that the quality of ε-caprolactam that is obtained by depolymerization of polyether polyurethane containing nylon 6 wasted fabric (without pre-treatment in a separation section, in which the nylon 6 and polyether polyurethane comprising material is separated into a nylon 6 rich stream and a polyether polyurethane rich stream) and subsequently concentrated and purified by distillation is very poor as it does not meet any of the required specifications for major polymerization applications.

COMPARATIVE EXPERIMENT 4

Depolymerization of polyether polyurethane containing nylon 6 wasted fabric and recovery of ε-caprolactam, purification by permanganate treatment and distillation.

Polyether polyurethane containing nylon 6 wasted fabric with a polyether polyurethane content of 8.15 +/- 0.05 wt.% (same feedstock as used in EXAMPLE 2) was used in this COMPARATIVE EXPERIMENT.

The pre-treatment and separation of nylon 6 and polyether polyurethane procedure of EXAMPLE 1 was followed. The recovered polyether polyurethane precipitate can be re-used as-such or in combination with virgin polyether polyurethane in the production of textiles. The depolymerization and recovery procedure of EXAMPLE 3 was followed.

The resulting crude ε-caprolactam was treated with 0.2 wt.% $KMnO_4$ with regard to ε-caprolactam at 50 °C for 2 hours. The solids formed were then removed from the oxidized reaction product by means of a filtration.

The ε-caprolactam in the oxidized reaction product was after addition of 75 mmol of aqueous sodium hydroxide per kg ε-caprolactam further purified by distillation as described in COMPARATIVE EXAMPLE 3.
The specifications of the distilled ε-caprolactam were:

| | |
|---|---|
| PAN: | 3 |
| E290: | 0.12 |
| VB: | 1.45 mmol/kg |
| Alkalinity: | 1.96 mmol/kg |

From this COMPARATIVE EXPERIMENT, it can be concluded that purification of crude ε-caprolactam by permanganate treatment succeeded by distillation is insufficient to meet all the required specifications for major polymerization applications as quantified above.

EXAMPLE 5

Depolymerization of polyether polyurethane containing nylon 6 wasted fabric and recovery of ε-caprolactam, purification by extraction, back-extraction, distillation and crystallization.

Polyether polyurethane containing nylon 6 wasted fabric with a polyether polyurethane content of 8.15 +/- 0.05 wt.% (same feedstock as used in EXAMPLE 2) was used in this EXAMPLE.

The pre-treatment and separation of nylon 6 and polyether polyurethane procedure of EXAMPLE 1 was followed. The recovered polyether polyurethane precipitate can be re-used as-such or in combination with virgin polyether polyurethane in the production of textiles. The depolymerization and recovery procedure of EXAMPLE 3 was followed.

76 gram of crude ε-caprolactam that was obtained was extracted one time with 100 grams and nine times with 50 grams of solvent mixture 4-methyl-2-pentanol (50 wt.%) / cyclohexane (50 wt.%) at 25 °C. The ten resulting ε-caprolactam phases comprising solvent mixtures were combined and subsequently concentrated by evaporation in a rotavap that was operated under vacuum (9.5 kPa; water bath temperature was ca. 65 °C) to an ε-caprolactam concentration of about 40 wt.% and then fresh cyclohexane was added. The ε-caprolactam concentration of the resulting mixture was about 25 wt.% and the weight ratio of the solvent mixture 4-methyl-2-pentanol / cyclohexane was 50 wt.% : 50 wt.%. Subsequently, the concentrated ε-caprolactam comprising solvent mixture was extracted eight times with 50 grams of water at 25 °C. The eight resulting aqueous ε-caprolactam phases were combined. The combined aqueous phases were concentrated by evaporation in a rotavap (rotary evaporator) that was operated under vacuum (9.5 kPa; water bath temperature was ca. 65 °C) to an ε-caprolactam concentration of 47.1 wt.%. The specifications of the obtained concentrated aqueous ε-caprolactam solution were:

| | |
|---|---|
| PAN: | 108 |
| E290: | 1.58 |

75 mmol of aqueous sodium hydroxide per kg ε-caprolactam was added to the obtained concentrated aqueous ε-caprolactam solution.

Subsequently, water and impurities with lower boiling points than ε-caprolactam were removed as top products by distillation under reduced pressure in a batch-wise operated distillation set-up. Finally, distilled ε-caprolactam was recovered as top product at 300 Pa, while the impurities with higher boiling points compared to ε-caprolactam remained as bottom product in the distillation set-up.

Then distilled water was added to the distilled ε-caprolactam to obtain a mixture with an ε-caprolactam concentration of 91.4 wt.%. This aqueous ε-caprolactam was introduced in a crystallization set-up at a temperature of 52 °C. The aqueous ε-caprolactam was cooled down to 40 °C and then 0.016 grams seeds were added to the mixture. Thereafter, the cooling of the mixture continued down to 30 °C, where it was held for 30 minutes. The crystallized ε-caprolactam was recovered by filtration and washed with an 85 wt.% aqueous ε-caprolactam solution. The specifications of the obtained purified ε-caprolactam were:

| | |
|---|---|
| PAN: | 2 |
| E290: | 0.02 |
| VB: | < 0.01 mmol/kg |
| Alkalinity: | 0.03 mmol/kg |

From this EXPERIMENT, it can be concluded that purified ε-caprolactam that meets all the required specifications for major polymerization applications can be obtained from depolymerization of nylon 6 that originates from polyether polyurethane containing nylon 6 wasted fabric and was purified by extraction, back-extraction, distillation and crystallization.

## EXAMPLE 6

### Pre-treatment and separation of nylon 6 and polyether polyurethane, depolymerization of nylon 6 and recovery of ε-caprolactam, purification by extraction, back-extraction, distillation and crystallization.

The pre-treatment and separation of nylon 6 and polyether polyurethane procedure of EXAMPLE 1 was followed. The recovered polyether polyurethane precipitate can be re-used as-such or in combination with virgin polyether polyurethane in the production of textiles. The depolymerization and recovery procedure of EXAMPLE 3 was followed.

36 gram of crude ε-caprolactam that was obtained was extracted one time with 68 gram and four times with 50 gram benzene at 25 °C. The resulting organic extracts were combined and concentrated by evaporation in a rotavap that was operated under vacuum (9.5 kPa; water bath temperature was ca. 65 °C) to an ε-caprolactam concentration of about 25 wt.% This mixture was 2 times batch-wise extracted with 25 gram water at a temperature of ca. 25 °C. The specifications of the aqueous ε-caprolactam solution after back-extraction were:

| | |
|---|---|
| PAN: | 132 |
| E290: | 1.55 |

75 mmol of aqueous sodium hydroxide per kg ε-caprolactam was then added to the concentrated ε-caprolactam solution. Subsequently, water and impurities with lower boiling points than ε-caprolactam were removed as top products by distillation under reduced pressure in a batch-wise operated distillation set-up. Finally, distilled ε-caprolactam was recovered as top product at 300 Pa, while the impurities with higher boiling points compared to ε-caprolactam remained as bottom product in the distillation set-up.

Then distilled water was added to the distilled ε-caprolactam to obtain a mixture with an ε-caprolactam concentration of 91.4 wt.%. This aqueous ε-caprolactam was introduced in a crystallization set-up at a temperature of 52 °C. The aqueous ε-caprolactam was cooled down to 40 °C and some seeds were added to the mixture. Thereafter, the cooling of the mixture continued down to 30 °C, where it was held for 30 minutes. The crystallized ε-caprolactam was recovered by filtration and washed with an 85 wt.% aqueous ε-caprolactam solution. The specifications of the obtained purified ε-caprolactam fulfilled all of the required specifications for major polymerization applications.

This EXAMPLE shows that polyether polyurethane and nylon 6 can be separated from polyether polyurethane containing nylon 6 wasted fabric by selective extraction, precipitation of the dissolved polyether polyurethane and washing of the non-dissolved nylon 6. Further, from this EXPERIMENT, it can be concluded that purified ε-caprolactam that meets all the required specifications for major polymerization applications can be obtained by depolymerization of nylon 6 that originates from discarded polyether polyurethane containing nylon 6 wasted fabric and was purified by extraction, back-extraction, distillation and crystallization.

EXPERIMENT 7

Calculation of carbon footprint of purified ε-caprolactam and polyether polyurethane.

A continuous process according to the invention for the production of purified ε-caprolactam and polyether polyurethane from polyether polyurethane containing nylon 6 wasted fabric was simulated. The polyether polyurethane content of these polyether polyurethane containing nylon 6 wasted fabric was 20 wt.%, the remainder mainly being nylon 6. The process included:

- Cutting polyether polyurethane containing nylon 6 wasted fabric in small pieces;
- Selective extraction of polyether polyurethane with DMAc;
- Separation of non-dissolved nylon 6 and polyether polyurethane rich stream by centrifugation;
- Washing of non-dissolved nylon 6 with water;
- Separation of washed non-dissolved nylon 6 and aqueous extract by centrifugation;
- Precipitation of polyether polyurethane from the polyether polyurethane rich stream by addition of the aqueous extract obtained above;
- Separation of precipitated polyether polyurethane by filtration from DMAc-water mixture;
- Recovery of DMAc and water from DMAc-water mixture obtained above;
- Drying of filtered polyether polyurethane;
- Drying of washed non-dissolved nylon 6;
- Melting and pelletization of washed non-dissolved nylon 6;
- Depolymerization of nylon 6 under influence of $H_3PO_4$ and superheated steam;
- Recovery of crude ε-caprolactam (80 wt.% ε-caprolactam) by partial condensation of vapors discharged from depolymerization reactor;
- Counter-current extraction of concentrated crude ε-caprolactam with toluene;
- Washing of organic extract with diluted caustic solution;
- Counter-current back-extraction of washed organic extract with water;
- Evaporative concentration of aqueous extract;
- Addition of caustic;
- Distillative removal of lights and heavies by vacuum distillation; and
- Recovery of pure ε-caprolactam by melt crystallization at a temperature of 61 °C.

The carbon footprints of purified ε-caprolactam and polyether polyurethane were calculated based on the consumption figures of raw materials, and utilities of the above described process are based on data originating from ecoinvent version 3.7.1. The distribution of the environmental impact between the products purified ε-caprolactam and polyether polyurethane in the pre-treatment section and in the separation section was based on the weight ratio of these products.

The outcome revealed that the product carbon footprint of purified ε-caprolactam obtained from polyether polyurethane containing polyamide 6 wasted fabric is less than 3.0 ton $CO_2$ eq. per ton of ε-caprolactam and less than 1.0 ton $CO_2$ eq. per ton of polyether polyurethane (location Europe).

While the invention has been described and with reference to specific embodiments thereof, it will be apparent to those of ordinary skill in the art that various changes and modifications, including (semi-)continuous operations and upscaling to commercial scale, can be made therein without departing from the spirit and scope thereof.

## Claims

**1.** A process for recovering ε-caprolactam and polyether polyurethane from a nylon 6 and polyether polyurethane comprising material in a plant, wherein the plant comprises

- a separation section [B],
- a depolymerization section [C],
- a recovery section [D], and
- a purification section [E],

and wherein the process comprises the steps of:

a) charging the nylon 6 and polyether polyurethane comprising material to the separation section [B];

b) separating the nylon 6 and polyether polyurethane comprising material in a nylon 6 rich stream and a polyether polyurethane rich stream in the separation section [B] by selective dissolution of the polyether polyurethane in an organic solvent at a temperature below 100 °C, preferably at a temperature ranging from 0 °C to 100 °C, more preferably at a temperature ranging from 10 °C to 90 °C, even more preferably at a temperature ranging from 10 °C to 80 °C, and most preferably at a temperature ranging from 20 °C to 75 °C, wherein the polyether polyurethane rich stream is a solution comprising the organic solvent and polyether polyurethane;

c.1) discharging the nylon 6 rich stream from separation section [B] and charging the nylon 6 rich stream to depolymerization section [C] wherein the nylon 6 content of the nylon 6 rich stream is at least 85 % by weight on dry-weight basis;

c.2) depolymerizing the nylon 6 in the nylon 6 rich stream in the depolymerization section [C] at a temperature ranging from 180 °C to 400 °C, preferably from 200 °C to 350 °C, more preferably from 220 °C to 340 °C, and most preferably from 240 °C to 325 °C so that an ε-caprolactam comprising stream is obtained and discharging the obtained ε-caprolactam comprising stream from the depolymerization section [C];

c.3) recovering crude ε-caprolactam from said ε-caprolactam comprising stream in the recovery section [D];

c.4) purifying the crude ε-caprolactam obtained in the recovery section [D] in the purification section [E] to obtain purified ε-caprolactam wherein the purification comprises the steps of

(iv) obtaining purified ε-caprolactam by crystallization of ε-caprolactam from a solution comprising ε-caprolactam and impurities at a temperature of 10 °C to 95 °C.

d.1) recovering polyether polyurethane from the polyether polyurethane rich stream in the separation section [B]; and

d.2) discharging said recovered polyether polyurethane from the separation section [B] wherein the polyether polyurethane content of the discharged stream is at least 85 % by weight on dry-weight basis.

**2.** A process according to claim 1, wherein in the purification section [E] the purification further comprises the additional steps of:
c.4)

(i) extracting the crude ε-caprolactam with an organic solvent so that an aqueous phase and an organic phase are obtained, and wherein the organic phase comprises the organic solvent, ε-caprolactam and impurities, and preferably the organic solvent is selected from the group consisting of cyclohexane, benzene, toluene, methylene chloride, chloroform, trichloroethane, 4-methyl-2-pentanol, 1-octanol, 2-ethylhexanol and mixtures thereof; and optionally the organic phase obtained is washed with water or with an aqueous alkaline solution;

(ii) optionally, switching the solvent by replacing the organic solvent at least partially with water or an aqueous solution, whereby an aqueous phase comprising water, ε-caprolactam and impurities with lower- or higher-boiling points than ε-caprolactam is obtained and wherein the solvent switch is selected from a process based on back-extraction with water, and a process based on solvent swap distillation, in which the organic solvent is distilled off and water is charged; and

(iii) optionally, obtaining purified ε-caprolactam by distillative removal of impurities with lower- or higher-boiling points than ε-caprolactam.

**3.** A process according to claim 1 or 2, wherein the separation section [B] comprises:

- a dissolution section [α],
- a washing section [β],
- a precipitation section [γ], and
- a solvent distillation section [δ],

and wherein the process comprises the following steps in the separation section [B]:

b.1) charging an organic solvent, and the nylon 6 and polyether polyurethane comprising material to the dissolution section [α];

b.2) dissolving polyether polyurethane from the nylon 6 and polyether polyurethane comprising material in an organic solvent in the dissolution section [α], so that a polyether polyurethane rich stream comprising organic solvent and dissolved polyether polyurethane, and a stream comprising non-dissolved nylon 6 are obtained and discharging the obtained streams from the dissolution section [α];

b.3) charging a second solvent and said polyether polyurethane rich stream comprising organic solvent and dissolved polyether polyurethane to the precipitation section [γ] so that polyether polyurethane is precipitated

from a mixture comprising organic solvent and second solvent;

b.4) recovering said precipitated polyether polyurethane from said mixture comprising organic solvent and second solvent and discharging said precipitated polyether polyurethane from the precipitation section [γ];

b.5) discharging the mixture comprising organic solvent and second solvent from which precipitated polyether polyurethane has been recovered in step b.4) from the precipitation section [γ] and charging said mixture to the solvent distillation section [δ];

b.6) charging a third solvent and the stream comprising non-dissolved nylon 6 to the washing section [β];

b.7) washing the stream comprising non-dissolved nylon 6 with the third solvent in the washing section [β], so that a nylon 6 rich stream and a mixture comprising organic solvent and third solvent are obtained;

b.8) discharging said nylon 6 rich stream from the washing section [β];

b.9) discharging the mixture comprising organic solvent and third solvent that is obtained in step b.7) from the washing section [β] and charging said mixture partly or completely to the solvent distillation section [δ];

b.10) separating organic solvent from the second solvent and the third solvent by distillation in the solvent distillation section [δ] and discharging the second solvent, the third solvent, and the separated organic solvent from the solvent distillation section [δ].

**4.** A process according to claim 3, wherein the separated organic solvent that is discharged from the solvent distillation section [δ] in step b.10) is charged to the dissolution section [α] in step b.1).

**5.** A process according to any one of claims 1 to 4, wherein

(i) the nylon 6 rich stream that is discharged from the separation section [B] is dried and/or densified prior to being charged to the depolymerization section [C]; and/or

(ii) the depolymerization in step c.2) is performed in the presence of water so that the ε-caprolactam comprising stream is a vapor stream comprising ε-caprolactam and water in a weight to weight ratio of from 1:1 to 1:50, in particular from 1:2 to 1:15, from 1:2 to 1:10 or from 1:3 to 1:8; and/or

(iii) the depolymerization section [C] is charged with superheated steam having a temperature ranging from 220 °C to 575 °C, in particular from 275 °C to 500 °C.

(iv) the depolymerization in step c.2) is carried out in the absence or presence of a catalyst, wherein the catalyst is selected from an acid and a base catalyst, the acid catalyst being selected from the group consisting of orthophosphoric acid, boric acid, sulfuric acid, organic acid, organic sulfonic acid, solid acids, salts of the aforementioned acids, $Al_2O_3$ and $SiO_2$, and combinations thereof, in particular orthophosphoric acid, and the base catalyst being selected from the group consisting of alkali hydroxide, alkali salt, alkaline earth hydroxide and alkaline earth salts, organic bases and solid bases, and combinations thereof, in particular, sodium hydroxide, potassium hydroxide, sodium carbonate, potassium carbonate, sodium hydrogen carbonate, and potassium hydrogen carbonate, preferably in the absence of a catalyst or in the presence of orthophosphoric acid.

**6.** A process according to any one of claims 3 to 5, wherein the recovered polyether polyurethane that is discharged from the precipitation section [γ] in step b.4) is re-used in the production of textiles.

**7.** A process according to any one of claims 1 to 6, wherein the plant further comprises

- a pre-treatment section [A],

and wherein prior to step a) the nylon 6 and polyether polyurethane comprising material is subjected to a pre-treatment in the pre-treatment section [A], in particular to a cleaning in a cleaning section [ω] and/or to a mechanical size reduction in a mechanical size reduction section [λ].

**8.** A process according to any one of claims 1 to 7, wherein the organic solvent is dimethylacetamide and, optionally, wherein the second solvent and the third solvent are the same solvent, preferably an aqueous solution or water.

**9.** A process according to any one of claims 1 to 8, wherein the second solvent in step b.3) is partly or completely the mixture comprising organic solvent and third solvent that is obtained in step b.7) from the washing section [β].

**10.** A process according to any one of claims 1 to 9, wherein the second solvent and the third solvent that are discharged from the solvent distillation section [δ], optionally after separation from each other, are re-used in the precipitation section [γ] and/or in the washing section [β].

**11.** A process according to any one of claims 4 to 10, wherein degradation products of the organic solvent are removed prior to charging the separated organic solvent that is discharged from the solvent distillation section [δ] in step b.10) to the dissolution section [α] in step b.1).

**12.** A process according to any one of claims 1 to 11, wherein the solution comprising ε-caprolactam and impurities from which ε-caprolactam is crystallized in step c.4)(iv) also comprises water, preferably more than 1 % by weight.

**13.** A plant for the production of purified ε-caprolactam and polyether polyurethane from nylon 6 and polyether polyurethane comprising material, wherein the plant comprises

- optionally, a pre-treatment section [A],
- a separation section [B],
- a nylon 6 depolymerization section [C],
- an ε-caprolactam recovery section [D],
- an ε-caprolactam purification section [E], and

wherein the chemical plant is configured for carrying out a process as defined in any one of claims 1 to 12.

**14.** Polyether polyurethane obtained via a process as defined in any one of claims 1 to 12, wherein the polyether polyurethane has a product carbon footprint of less than 1.0 kg $CO_2$ eq. per kg recovered polyether polyurethane.

**15.** Purified ε-caprolactam obtained via a process as defined in any one of claims 1 to 12, wherein the ε-caprolactam has a product carbon footprint of less than 3.0 kg $CO_2$ eq. per kg purified ε-caprolactam.

FIG. 1

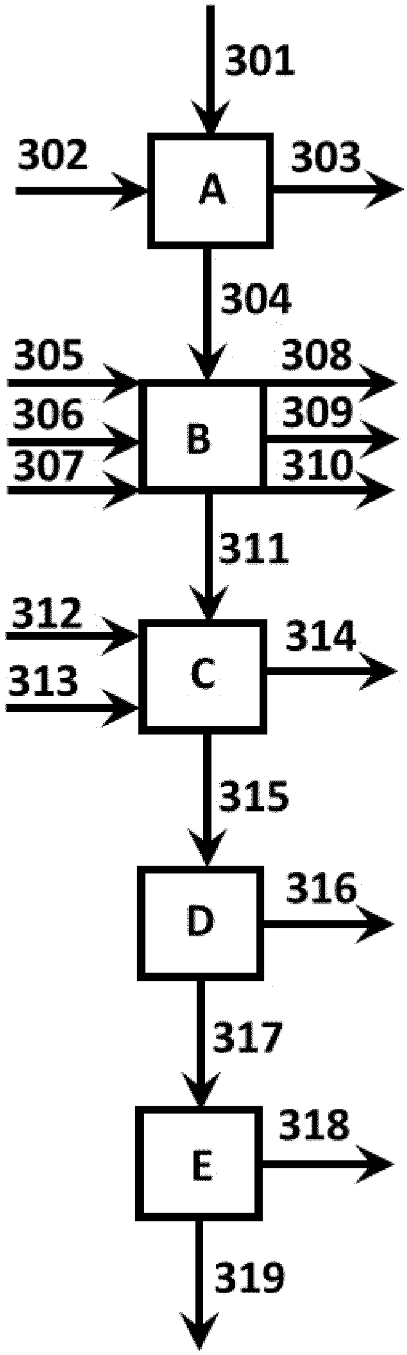
301
302
A
303
304
305
306
307
B
308
309
310
311
312
313
C
314
315
D
316
317
E
318
319

FIG. 2A

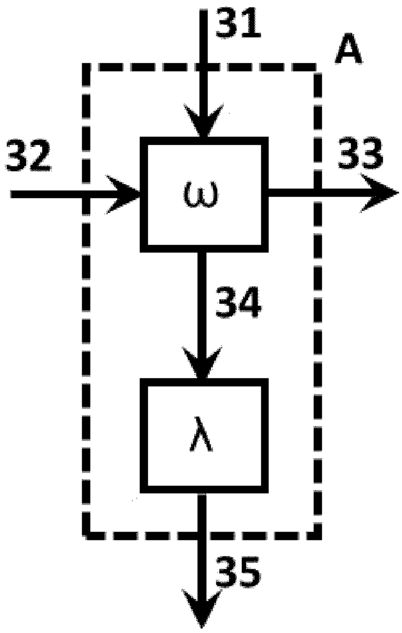


FIG. 2B

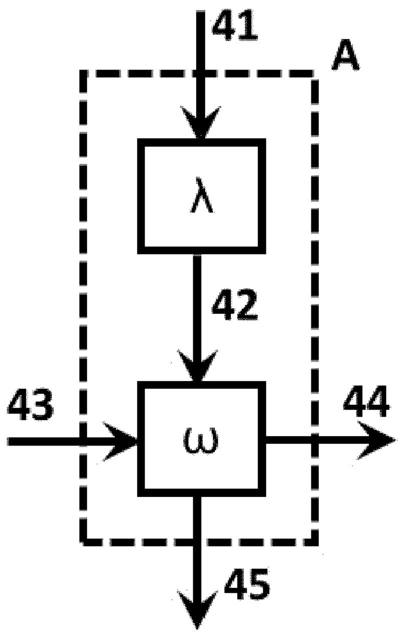

FIG. 3

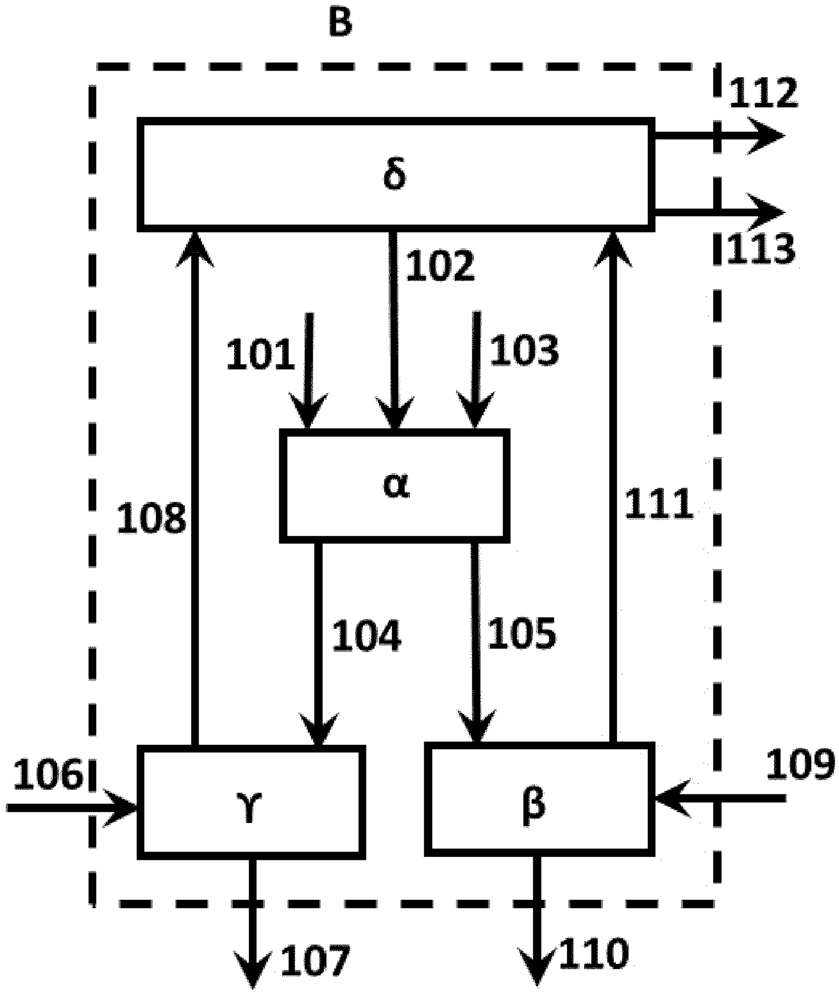

FIG. 4A

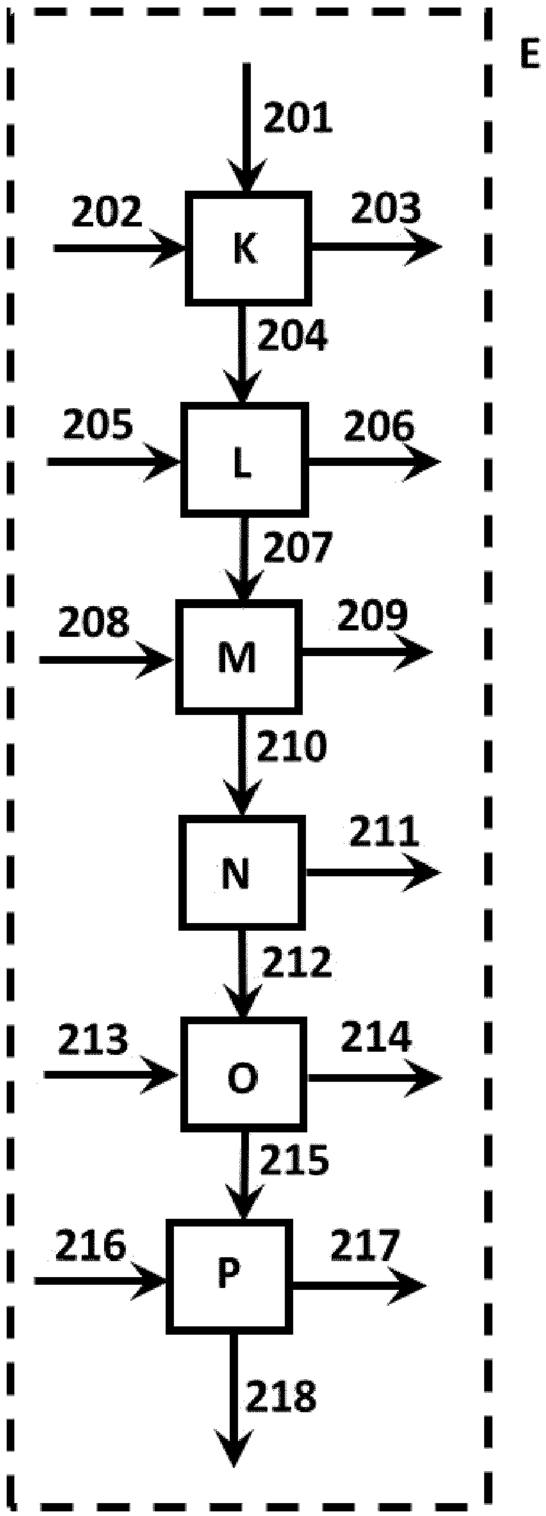
E
201
202
K
203
204
205
L
206
207
208
M
209
210
N
211
212
213
O
214
215
216
P
217
218

FIG. 4B

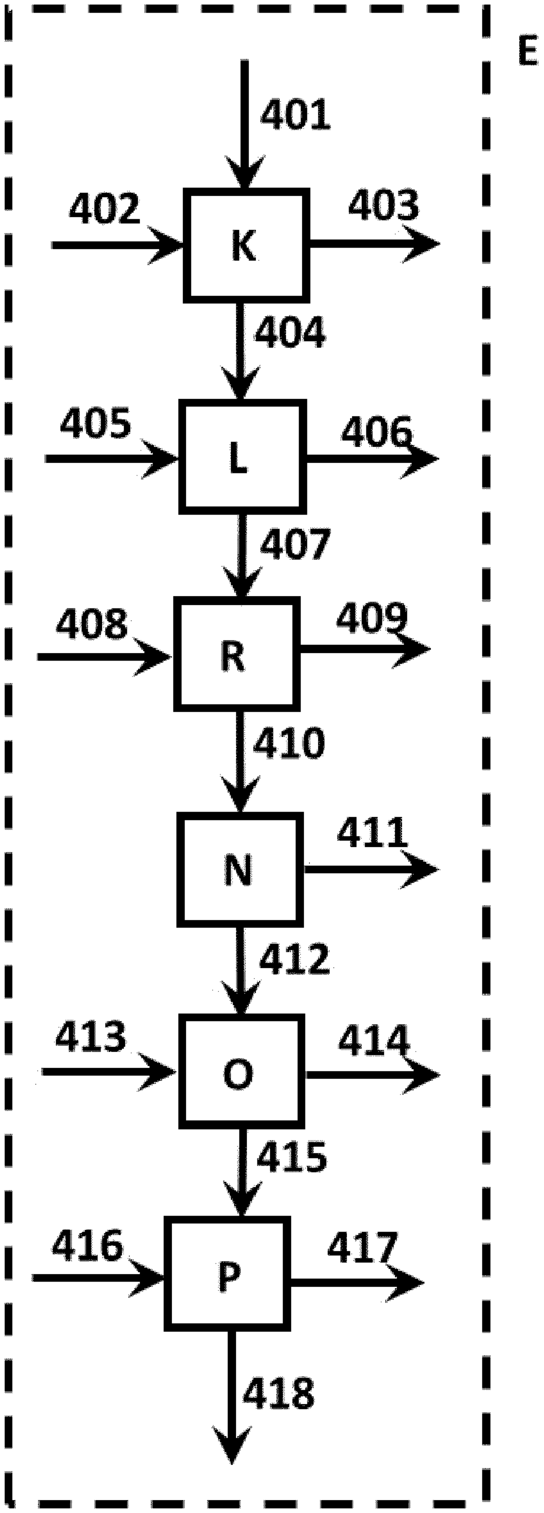
E
401
402
K
403
404
405
L
406
407
408
R
409
410
N
411
412
413
O
414
415
416
P
417
418

Europäisches Patentamt
European Patent Office
Office européen des brevets

# EUROPEAN SEARCH REPORT

Application Number
EP 22 18 4110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GONG CAIHONG ET AL: "Simple process for separation and recycling of nylon 6 and polyurethane components from waste nylon 6/polyurethane debris", TEXTILE RESEARCH JOURNAL, vol. 91, no. 1-2, 17 June 2020 (2020-06-17), pages 18-27, XP93006676, GB ISSN: 0040-5175, DOI: 10.1177/0040517520931893 Retrieved from the Internet: URL:http://journals.sagepub.com/doi/full-xml/10.1177/0040517520931893> | 13-15 | INV. C08J11/08 C08J11/12 C08J11/14 |
| Y | * Title, Scheme 1, page 19, right, lines 11-13 and lines 17-18, Figure 4 (IR) for nylon and Figure 6 (NMR) for the urethane, page 23, right, lines 37-39. * ----- | 1-12 | |
| Y | YIN YUNJIE ET AL: "Removal of spandex from nylon/spandex blended fabrics by selective polymer degradation", TEXTILE RESEARCH JOURNAL, vol. 84, no. 1, 21 May 2013 (2013-05-21), pages 16-27, XP93006635, GB ISSN: 0040-5175, DOI: 10.1177/0040517513487790 Retrieved from the Internet: URL:http://journals.sagepub.com/doi/full-xml/10.1177/0040517513487790> * p17, left, lines 14-18 * ----- | 8 | TECHNICAL FIELDS SEARCHED (IPC) C08J C07D |
| A | WO 2021/021031 A1 (LAI TRILLION [TH]) 4 February 2021 (2021-02-04) * the whole document * ----- -/-- | 1-15 | |

The present search report has been drawn up for all claims

| Place of search | Date of completion of the search | Examiner |
|---|---|---|
| The Hague | 23 December 2022 | Mooibroek, Tiddo |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 22 18 4110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/115602 A1 (REGENERATED TEXTILE IND LLC [US]) 2 June 2022 (2022-06-02)<br>* the whole document *<br>----- | 1-15 | |
| A | US 2014/255255 A1 (HEILBERG RONALDO DANIEL [BR]) 11 September 2014 (2014-09-11)<br>* the whole document *<br>----- | 1-15 | |
| A | JP 2011 088943 A (TORAY INDUSTRIES) 6 May 2011 (2011-05-06)<br>* the whole document *<br>----- | 1-15 | |
| A | US 6 830 715 B1 (REINEHR ULRICH [DE] ET AL) 14 December 2004 (2004-12-14)<br>* the whole document *<br>----- | 1-15 | |
| A | CA 771 086 A (DU PONT) 7 November 1967 (1967-11-07)<br>* the whole document *<br>----- | 1-15 | |
| Y | US 5 948 908 A (SIFNIADES STYLIANOS [US] ET AL) 7 September 1999 (1999-09-07)<br>* column 6, line 40 - line 49; claims 1, 2, 6-8 *<br>----- | 1-7,9-12 | TECHNICAL FIELDS SEARCHED (IPC) |
| A | WO 2022/129022 A1 (BASF SE [DE]) 23 June 2022 (2022-06-23)<br>* the whole document *<br>----- | 1-15 | |
| A | EP 0 670 308 A1 (BASF AG [DE]) 6 September 1995 (1995-09-06)<br>* column 1, line 23 - line 34 *<br>----- | 1-15 | |
| A | US 5 990 306 A (MAYER RICHARD EUGENE [US] ET AL) 23 November 1999 (1999-11-23)<br>* the whole document *<br>-----<br>-/-- | 1-15 | |

The present search report has been drawn up for all claims

1

| Place of search | Date of completion of the search | Examiner |
|---|---|---|
| The Hague | 23 December 2022 | Mooibroek, Tiddo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 22 18 4110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 173 419 A1 (DSM NV [NL]) 23 January 2002 (2002-01-23) * the whole document * ----- | 1-15 | |

TECHNICAL FIELDS SEARCHED (IPC)

The present search report has been drawn up for all claims

1

| Place of search | Date of completion of the search | Examiner |
|---|---|---|
| The Hague | 23 December 2022 | Mooibroek, Tiddo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 22 18 4110

This annex lists the patent family members relating to the patent documents cited in the above-mentioned European search report. The members are as contained in the European Patent Office EDP file on
The European Patent Office is in no way liable for these particulars which are merely given for the purpose of information.

23-12-2022

| Patent document cited in search report | Publication date | Patent family member(s) | Publication date |
|---|---|---|---|
| WO 2021021031 A1 | 04-02-2021 | NONE | |
| WO 2022115602 A1 | 02-06-2022 | NONE | |
| US 2014255255 A1 | 11-09-2014 | BR PI1104721 A2 | 18-08-2015 |
| | | EP 2596932 A2 | 29-05-2013 |
| | | US 2014255255 A1 | 11-09-2014 |
| | | WO 2013075184 A1 | 30-05-2013 |
| JP 2011088943 A | 06-05-2011 | NONE | |
| US 6830715 B1 | 14-12-2004 | AU 3154500 A | 14-09-2000 |
| | | BR 0008485 A | 29-01-2002 |
| | | CA 2362175 A1 | 31-08-2000 |
| | | CN 1341170 A | 20-03-2002 |
| | | DE 19907830 A1 | 31-08-2000 |
| | | EP 1163381 A1 | 19-12-2001 |
| | | HK 1045179 A1 | 15-11-2002 |
| | | JP 2002538314 A | 12-11-2002 |
| | | KR 20010102353 A | 15-11-2001 |
| | | MX PA01008560 A | 09-04-2002 |
| | | TW 469305 B | 21-12-2001 |
| | | US 6830715 B1 | 14-12-2004 |
| | | WO 0050673 A1 | 31-08-2000 |
| CA 771086 A | 07-11-1967 | NONE | |
| US 5948908 A | 07-09-1999 | AT 201015 T | 15-05-2001 |
| | | DE 69704767 T2 | 27-02-2003 |
| | | EP 0892782 A1 | 27-01-1999 |
| | | US 5869654 A | 09-02-1999 |
| | | US 5948908 A | 07-09-1999 |
| | | WO 9734868 A1 | 25-09-1997 |
| WO 2022129022 A1 | 23-06-2022 | NONE | |
| EP 0670308 A1 | 06-09-1995 | CA 2143820 A1 | 05-09-1995 |
| | | DE 4407222 A1 | 07-09-1995 |
| | | EP 0670308 A1 | 06-09-1995 |
| | | JP H0841021 A | 13-02-1996 |
| | | US 5700358 A | 23-12-1997 |
| US 5990306 A | 23-11-1999 | AT 368646 T | 15-08-2007 |
| | | CA 2302787 A1 | 11-03-1999 |
| | | CN 1278792 A | 03-01-2001 |
| | | EP 1042284 A1 | 11-10-2000 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 18 4110

This annex lists the patent family members relating to the patent documents cited in the above-mentioned European search report. The members are as contained in the European Patent Office EDP file on
The European Patent Office is in no way liable for these particulars which are merely given for the purpose of information.

23-12-2022

| Patent document cited in search report | Publication date | Patent family member(s) | Publication date |
|---|---|---|---|
| | | JP 2001514251 A | 11-09-2001 |
| | | KR 20010023618 A | 26-03-2001 |
| | | TW I225482 B | 21-12-2004 |
| | | US 5990306 A | 23-11-1999 |
| | | US 6187917 B1 | 13-02-2001 |
| | | WO 9911616 A1 | 11-03-1999 |
| EP 1173419 A1 | 23-01-2002 | EP 1173419 A1 | 23-01-2002 |
| | | US 2002038023 A1 | 28-03-2002 |
| | | WO 0064871 A1 | 02-11-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6830715 B1
- JP 2011088943 A
- WO 2013032408 A1
- EP 635487 A

### Non-patent literature cited in the description


- *CHEMICAL ABSTRACTS,* 25038-54-4
- Caprolactam. Ullmann's Encyclopedia of Industrial Chemistry. Wiley-VCH Verlag GmbH & Co. KGaA, 25 May 2018
- Polyamides. Ullmann's Encyclopedia of Industrial Chemistry. Wiley-VCH Verlag GmbH & Co. KGaA, 15 January 2013
- **J. HONG ; X. XU.** Environmental impact assessment of caprolactam production - a case study in China. *J. of Cleaner production,* 2012, vol. 27, 103-108
- **N.M. VAN DER VELDEN ; M.K. PATEL ; J.G. VOGTLÄNDER.** LCA benchmarking study on textiles made of cotton, polyester, nylon, acryl, or elastane. *Int J Life Cycle Assess,* 2014, vol. 19, 331-356